# EUROPEAN PATENT APPLICATION

(11) **EP 2 098 231 A1**
(43) Date of publication of application: **09.09.2009**
(21) Application number: 08102310.3
(22) Date of filing: 05.03.2008
(51) Int. Cl.: A61K 31/44, A61P 9/10

(54) **Use of NAD formation inhibitors for the treatment of ischemia-reperfusion injury**

(71) Applicant: Topotarget Switzerland SA, 1004 Lausanne (CH)
(72) Inventor: Leo, Oberdan, 1970, WEZEMBEEK-OPPEM (BE); van Gool, Frédéric, 1950, KRAAINEM (BE); Galli, Mara, 6000, CHARLEROI (BE); Flamand, Véronique, 1030, BRUXELLES (BE); Loi, Patrizia, 6210, MELLET (BE); de Smedt, Thibaut, 1009, PULLY (CH)
(74) Representative: Tetaz, Franck Claude Edouard

(57) **Abstract**

This invention generally relates to the use of inhibitors of the enzyme nicotinamide phosphoribosyltransferase NAMPRTase for the prevention and/or treatment of ischemia-reperfusion injury and reperfusion injury associated with a variety of disease and conditions.

## Description

### Field of the invention

This invention generally relates to the use of inhibitors of the enzyme nicotinamide phosphoribosyltransferase NAMPRTase for the prevention and/or treatment of ischemia-reperfusion injury and reperfusion injury associated with a variety of disease and conditions.

### Background

The preparation of a class of compounds, comprising several subclasses, which act as inhibitors of the formation of nicotinamide adenine nucleotide, and their use thereof as anti-tumour agents, is already described in the patent applications WO0050399,WO97/48695, WO97/48397, WO99/31063, W09931060, W09931087, WO9931064,WO00/50399, and WO0380054. Especially useful compounds are described in the PCT application W09748696.

One of these inhibitors, (E)-N-[4-(1-benzoylpiperidin-4-yl) butyl]-3-(pyridine-3-yl)-acrylamide also known as AP0866, FK866, WK175, or WK22.175 and hereinafter referred to as FK866 [International Non-proprietary Name], is especially described in the literature as an anticancer agent.

FK866 may be used for treatment of diseases implicating deregulated apoptosis such as cancer. It has been demonstrated in the prior art that FK866 interferes with nicotinamide adenine dinucleotide (also known and hereinafter referred to as NAD) biosynthesis and induces apoptotic cell death without any DNA damaging effects.

Antiangiogenic and antitumoral efficacy of FK866 are described in many publications.

The publication "FK866, a high specific non-competitive inhibitor of nicotinamide phosphoribosyltransferase, represents a novel mechanism for induction of tumor cell apoptosis", M. Hasmann et al., Cancer Research 63, 7436-7442, November 1, 2003 describes more generally FK866 as the first high potent and specific inhibitor of nicotinamide phosphoribosyltransferase and its characteristics as antitumor compound. For example, its efficacy as antitumor agent for the treatment of leukaemia is described in "WK175, a novel antitumor agent, decreases the intracellular nicotinamide adenine dinucleotide concentration and induces the apoptotic cascade in human leukaemia cells", K. Wosikowski et al., Cancer Research 62, 1057-1062, February 15, 2002. Ischemia-reperfusion (I/R) injury refers to damage to a tissue caused when the blood supply returns to the tissue after a period of ischemia (restriction in blood supply). The absence of oxygen and nutrients from the blood creates a condition in which the restoration of circulation of circulation results in inflammation and oxidative damage, rather than restoration of normal function. Ischemia-reperfusion injury can be associated with traumatic injury, including hemorrhagic shock, as well as many other medical conditions such as stroke, or large vessel occlusion, and is a major medical problem. More particularly, ischemia reperfusion injury is important in heart attacks, stroke, kidney failure following vascular surgery, post-transplantation injury and chronic rejection as well as in various types of traumatic injury where hemorrhage will lead to organ hypoperfusion and then subsequent reperfusion injury during fluid resuscitation. Ischemia-reperfusion injury or an injury due to reperfusion and ischemic events is also observed in a variety of autoimmune and inflammatory diseases. Independently of other factors, ischemia-reperfusion injury leads to increased mortality.

### Description of the invention

In the present invention, unexpected properties of the inhibition of nicotinamide adenine dinucleotide (NAD) have been identified useful for the inhibition of ischemic-reperfusion injury.

This finding that inhibitors of the formation of NAD, for example inhibitors of the enzyme nicotinamide phosphoribosyltransferase, have activities which make them particularly suitable in an excellent manner for the prevention and/or treatment of ischemic-reperfusion injury was completely unexpected.

The present invention establishes a functional link between metabolism and ischemic-reperfusion injury, and demonstrates an important role for NAD dependent enzymes in the cellular damage caused during such injury.

Based on the results of the present invention, the present invention has important implication for design of novel treatment or prevention strategies for patients undergoing ischemia and reperfusion.

Hence in a first embodiment, the present invention relates to the use of an inhibitor of the formation of NAD for the preparation of a medicament used in the prevention or treatment of ischemia reperfusion injury.

In a second embodiment, the present invention relates to a process to manufacture a medicament for the prevention or treatment of ischemia-reperfusion injury.

In a third embodiment, the present invention also concerns a method of preventing or treating ischemia reperfusion injury comprising administering to a subject an effective amount of an inhibitor of the formation of NAD.

Furthermore, the present invention concerns also a pharmaceutical kit comprising at least an effective amount of an inhibitor of the formation of NAD together with instructions for use in the prevention or treatment of ischemia-reperfusion injury.

According to the present invention, the inhibitor is preferably a competitive, uncompetitive, non-competitive or mixed inhibitor of the enzyme nicotinamide phosphoribosyltransferase.

According to the present invention, the inhibitor is preferably a compound of formula (I) 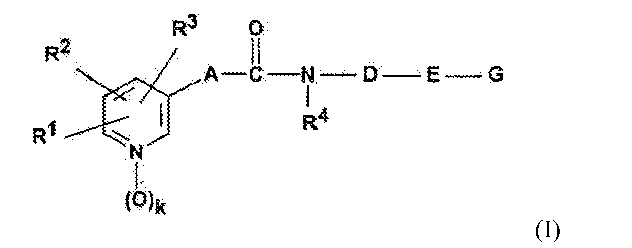 wherein :
- **R¹**: is selected from the group consisting of hydrogen, halogen, cyano, C₁-C₆- alkyl, trifluoromethyl, C₃-C₈-cycloalkyl, C₁-C₄-hydroxyalkyl, hydroxy, C₁- C₄-alkoxy, benzyloxy, C₁-C₄-alkanoyloxy, C₁-C₄-alkylthio, C₂-C₅- alkoxycarbonyl, aminocarbonyl, C₃-C₉-dialkylaminocarbonyl, carboxy, phenyl, phenoxy, pyridyloxy, and **NR⁵R⁶,** wherein
- **R⁵**: and
- **R⁶**: are selected independently from each other from hydrogen and C₁-C₆-alkyl,
- **R²**: is selected from hydrogen, halogen, C₁-C₆-alkyl, trifluoromethyl and hydroxy, wherein
- **R¹**: and **R²,** in the case they are adjacent, optionally form a bridge which is selected from the group of bridge members -(CH₂)₄- and -(CH=CH)₂- and - CH₂O-**CR⁷R⁸**-O-, wherein
- **R⁷**: and
- **R⁸**: are independent from each other, hydrogen or C₁-C₆-alkyl,
- **R³**: is selected from hydrogen, halogen and C₁-C₆-alkyl,
- **R⁴**: is selected from hydrogen, C₁-C₆-alkyl, C₃-C₆-alkenyl, hydroxy, C₁-C₆- alkoxy and benzyloxy,
- **k**: is 0 or 1,
- **A**: is selected from C₂-C₆-alkenylene, which is optionally substituted one to three-fold by C₁- C₃-alkyl, hydroxy, fluorine, cyano, or phenyl, C₄-C₆-alkadienylene, which is optionally substituted once or twice by C₁- C₃-alkyl, fluorine, cyano, or phenyl, 1,3,5-hexatrienylene, which is optionally substituted by C₁-C₃-alkyl, fluorine, or cyano, and ethinylene,
- **D**: is selected from C₁-C₁₀-alkylene, optionally substituted once or twice by C₁-C₃-alkyl or hydroxy, C₂-C₁₀-alkenylene, optionally substituted once or twice by C₁-C₃-alkyl or hydroxy, wherein the double bond optionally is to ring E. C₃-C₁₀-alkinylene, optionally substituted once or twice by C₁-C₃-alkyl or hydroxy, and the group consisting of C₁-C₁₀-alkylene, C₂-C₁₀-alkenylene and C₃-C₁₀- alkinylene, in which one to three methylene units are isosterically replaced by O, S, **NR⁹**, CO, SO or SO₂, wherein **R⁹** is selected from hydrogen, C₁-C₃-alkyl, C₁-C₆-acyl and methanesulfonyl,
- **E**: is selected from 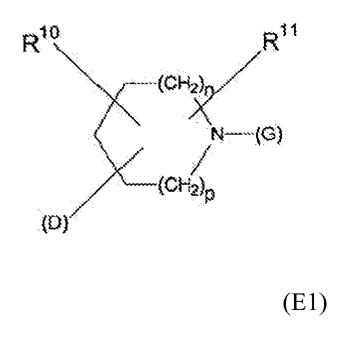 and 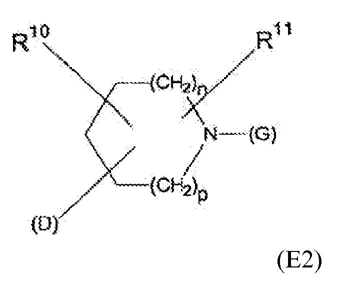 wherein the heterocyclic ring optionally has a double bond and
- **n** and **p**: are, independent of each other, 0, 1, 2 or 3, with the proviso that **n** + **p** ≤ 4,
- **q**: is 1 or 2,
- **R¹⁰**: is selected from hydrogen, C₁-C₃-atkyl, hydroxy, and hydroxymethyl, carboxy and C₂-C₇-alkoxycarbonyl,
- **R¹¹**: is hydrogen or an oxo group adjacent to the nitrogen atom,
- **G**: is selected from hydrogen, **G1, G2**, **G3**, **G4** and **G5**, wherein
- **G1**: represents the residue

**- (CH₂)ᵣ-(CR¹³R¹⁴)ₛ**-**R¹²** (G1)

wherein
- **r**: is 0, 1 or 2 and
- **s**: is 0 or 1,
- **R¹²**: is selected from hydrogen, C₁-C₆-alkyl, C₃-C₆-alkenyl, C₃-C₆-alkinyl, C₃- C₈-cycloalkyl, benzyl, phenyl, the group consisting of monocyclic aromatic five- and six-membered heterocycles, which contain one to three hetero-atoms selected from N, S and O and are either bound directly or over a methylene group, the group consisting of anellated bi- and tricyclic aromatic or partially hydrogenated carbocyclic ring systems with 8 to 16 ring atoms and at least one aromatic ring, wherein the bond occurs either over an aromatic or a hydrogenated ring and either directly or over a methylene group, and the group consisting of anellated bi- and tricyclic aromatic or partially hydrogenated heterocyclic ring systems with 8 to 16 ring atoms and at least one aromatic ring, wherein one to three ring atoms are selected from N, S and O and the bond occurs either over an aromatic or a hydrogenated ring, and either directly or over a methylene group,
- **R¹³**: has the same meaning as **R¹²,** but is selected independently thereof,
- **R¹⁴**: is selected from hydrogen, hydroxy, methyl, benzyl, phenyl, the group consisting of monocyclic aromatic five- and six-membered heterocycles, which contain one to three hetero-atoms selected from N, S and O and are bound either directly or over a methylene group, the group consisting of anellated bi- and tricyclic aromatic or partially hydrogenated carbocyclic ring systems with 8 to 16 ring atoms and at least one aromatic ring, wherein the bond occurs either over an aromatic or a hydrogenated ring and either directly or over a methylene group, and the group consisting of anellated bi- and tricyclic aromatic or partially hydrogenated heterocyclic ring systems with 8 to 16 ring atoms and at least one aromatic ring, wherein one to three ring atoms are selected from N, S and O and the bond occurs either over an aromatic or a hydrogenated ring and either directly or over a methylene group,
- **G2**: is selected from the residues 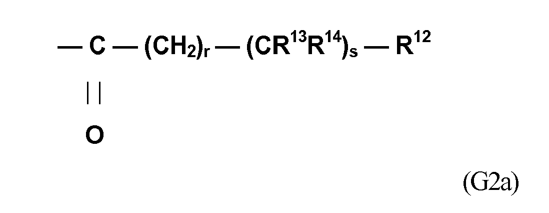 and 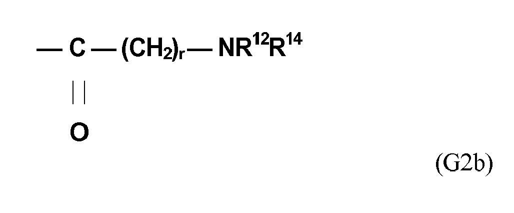 wherein the substituents **R¹²** and **R¹⁴** have the above meaning, or the group **-NR¹²R¹⁴** is a nitrogen-containing heterocycle bound over the nitrogen atom, the nitrogen-containing heterocycle being selected from the group consisting of saturated and unsaturated monocyclic, four- to eight- membered heterocycles, which, aside from the essential nitrogen atom, optionally contain one or two further hetero-atoms selected from N, S and O, and the group consisting of saturated and unsaturated bi- or tricyclic, anellated or bridged heterocycles with 8 to 16 ring atoms, which, aside from the essential nitrogen atom, optionally contain one or two further hetero-atoms selected from N, S and O,
- **G3**: is the residue

**-SO₂-(CH₂)ᵣR¹²,** (G3)
- **G4**: is the residue 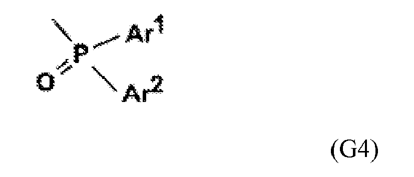
wherein
- **Ar¹**: and
- **Ar²**: are selected independently of each other from phenyl, pyridyl and naphthyl,
- **G5**: is the residue **-COR¹⁵** (G5) wherein
- **R¹⁵**: is selected from trifluoromethyl, C₁-C₆-alkoxy, C₃-C₆-alkenyloxy and benzyloxy,
wherein aromatic ring systems in the substituents **R¹, R², R⁴, R¹², R¹³, R¹⁴, R¹⁵, Ar¹** and **Ar²** and in the ring system -**NR¹²R¹⁴** optionally carry independently of each other one to three substituents which are independently selected from the group consisting of halogen, cyano, C₁-C₆-alkyl, trifluoromethyl, C₃-C₈-cycloalkyl, phenyl, benzyl, hydroxy, C₁-C₆-alkoxy, which is optionally entirely or partially substituted by fluorine, benzyloxy, phenoxy, mercapto, C₁-C₆-alkylthio, carboxy, C₁-C₆-alkoxycarbonyl, benzyloxycarbonyl, nitro, amino, mono-C₁-C₆-alkylamino, and di-(C₁-C₆-alkyl)-amino, wherein two adjacent groups of the aromatic ring or ring system optionally form an additional ring over a methylenedioxy bridge,
tautomeres in the case of substitution of the heterocycle or in an anellated ring system by free hydroxy, mercapto and/or amino groups, stereoisomers and/or mixtures thereof and pharmacologically acceptable acid addition salts with the exception of (E)-3-(3-pyridyl)-N-[2-(1-benzylpiperidin-4-yl)ethyl]-2-propenamide hydrochloride.

In a preferred embodiment the inhibitor is a compound of formula (I); wherein:
- **R¹**: is selected from hydrogen, halogen, cyano, methyl, trifluoromethyl, hydroxy, C₁-C₄-alkoxy, ethylthio, methoxycarbonyl, tert-butoxycarbonyl, aminocarbonyl, carboxy, and phenoxy,
- **R²**: is selected from hydrogen, halogen, trifluoromethyl and hydroxy,
- **R³**: is hydrogen or halogen,
- **R⁴**: is selected from hydrogen, C₁-C₃-alkyl, hydroxy and C₁-C₃-alkoxy,
- **k**: is 0 or 1,
- **A**: is selected from C₂-C₆-alkenylene, optionally substituted once or twice by C₁-C₃-alkyl, hydroxy or fluorine, C₄-C₆-alkadienylene, optionally substituted by C₁-C₃-alkyl or by 1 or 2 fluorine atoms, and 1, 3, 5-hexatrienylene, optionally substituted by fluorine,
- **D**: is selected from C₁-C₈-alkylene, optionally substituted once or twice by methyl or hydroxyl, C₂-C₈-alkenylene, optionally substituted once or twice by methyl or hydroxy, wherein the double bond optionally is to ring E, C₃-C₈-alkinylene optionally substituted once or twice by methyl or hydroxy, and the group consisting of C₁-C₈-alkylene, C₂-C₈-alkenylene and C₃-C₈- alkinylene in which one to three methylene units are isosterically replaced by O, S, NH, N(CH₃), N(COCH₃), N(SO₂CH₃) CO, SO or SO₂,
- **E**: is selected from 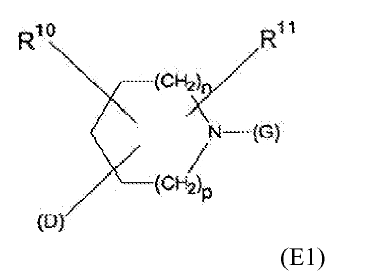 and 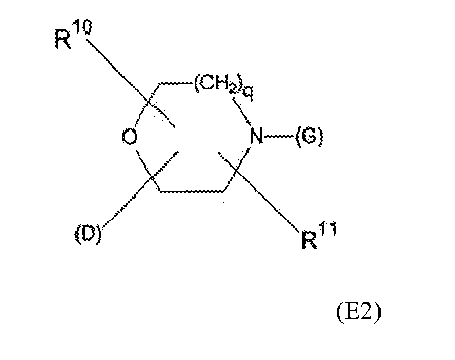 wherein the heterocyclic ring optionally has a double bond and
- **n**: and
- **p**: are, independent of each other, 0, 1, 2 or 3, with the proviso that n + **p** ≤ 3,
- **q**: is 1 or 2,
- **R¹⁰**: is selected from hydrogen, C₁-C₃-alkyl, hydroxy, and hydroxymethyl,
- **R¹¹**: is hydrogen or an oxo group which is adjacent to the nitrogen atom,
- **G**: is selected from hydrogen, **G1, G2**, **G3**, **G4** and **G5**, wherein
- **G1**: represents the residue

**-(CH2)ᵣ-(CR¹³R¹⁴)ₛ-R¹²** (G1)

wherein
- **r**: is 0, 1 or 2 and
- **s**: is 0 or 1,
- **R¹²**: is selected from hydrogen, C₁-C₆-alkyl, C₃-C₈-cycloalkyl, benzyl, phenyl, the group consisting of benzocyclobutyl, indanyl, indenyl, oxoindanyl, naphthyl, dihydronaphthyl, tetrahydronaphthyl, oxotetrahydronaphthyl, biphenylenyl, fluorenyl, oxofluorenyl, anthryl, dihydroanthryl, oxodihydroanthryl, dioxodihydroanthryl, phenanthryl, dihydrophenanthryl, oxodihydrophenanthryl, dibenzocycloheptenyl, oxodibenzocycloheptenyl, dihydrodibenzocycloheptenyl, oxodihydrodibenzocycloheptenyl, dihydrodibenzocyclooctenyl, tetrahydrodibenzocyclooctenyl and oxotetrahydrodibenzocyclooctenyl, bound directly or over a methylene group, and the group consisting of furyl, thienyl, pyrrolyl, oxazolyl, isoxazolyl, thiazolyl, isothiazolyl, pyrazolyl, imidazolyl, oxadiazolyl, thiadiazolyl, triazolyl, pyridyl, pyrazinyl, pyridazinyl, pyrimidinyl, triazinyl, imidazothiazolyl, benzofuryl, dihydrobenzofuryl, benzothienyl, dihydrobenzothienyl, indolyl, indolinyl, oxoindolinyl, dioxoindolinyl, benzoxazolyl, oxobenzoxazolinyl, benzisoxazolyl, oxobenzisoxazolinyl, benzothiazolyl, oxobenzthiazolinyl, benzoisothiazolyl, oxobenzoisothiazolinyl, benzimidazolyl, oxobenzimidazolinyl, indazolyl, oxoindazolinyl, benzofurazanyl, benzothiadiazolyl, benzotriazolyl, oxazolopyridyl, oxodihydrooxazolopyridyl, thiazolopyridyl, oxodihydrothiazolopyridyl, isothiazolopyridyl, imidazopyridyl, oxodihydroimidazopyridyl, pyrazolopyridyl, oxodihydropyrazolopyridyl, thienopyrimidinyl, chromanyl, chromanonyl, benzopyranyl, chromonyl, quinolyl, isoquinolyl, dihydroquinolyl, oxodihydroquinolinyl, tetrahydroquinolyl, oxotetrahydroquinolinyl, benzodioxanyl, quinoxalinyl, quinazolinyl, naphthyridinyl, carbazolyl, tetrahydrocarbazolyl, oxotetrahydrocarbazolyl, pyridoindolyl, acridinyl, oxodihydroacridinyl, phenothiazinyl, dihydrodibenzoxepinyl, oxodihydrodibenzoxepinyl, benzocycloheptathienyl, oxobenzocycloheptathienyl, dihydrothienobenzothiepinyl, oxodihydrothienobenzothiepinyl, dihydrodibenzothiepinyl, oxodihydrodibenzothiepinyl, octahydrodibenzothiepinyl, dihydrodibenzazepinyl, oxodihydrodibenzazepinyl, octahydrodibenzazepinyl, benzocycloheptapyridyl, oxobenzocycloheptapyridyl, dihydropyridobenzodiazepinyl, dihydrodibenzoxazepinyl, dihydropyridobenzoxepinyl, dihydropyridobenzoxazepinyl, oxodihydropyridobenzoxazepinyl, dihydrodibenzothiazepinyl, oxodihydrodibenzothiazepinyl, dihydropyridobenzothiazepinyl, and oxodihydropyridobenzothiazepinyl, bound directly or over a methylene group,
- **R¹³**: has the same meaning as **R¹²,** but is selected independently therefrom,
- **R¹⁴**: is selected from hydrogen, hydroxy, methyl, benzyl, phenyl, and, the group consisting of indanyl, indenyl, naphthyl, dihydronaphthyl, tetrahydronaphthyl, furyl, thienyl, pyrrolyl, oxazolyl, isoxazolyl, thiazolyl, isothiazolyl, pyrazolyl, imidazolyl, oxadiazolyl, thiadiazolyl, triazolyl, pyridyl, pyrazinyl, pyridazinyl, pyrimidinyl, triazinyl, benzofuryl, benzothienyl, indolyl, indolinyl, benzoxazolyl, benzothiazolyl, benzimidazolyl, chromanyl, quinolyl, and tetrahydroquinolyl, bound directly or over a methylene group,
- **G2**: is selected from the residue 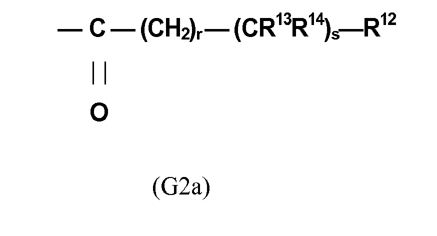 and 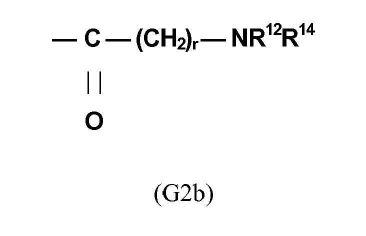 wherein the substituents **R¹²** and **R¹⁴** have the above meanings, or the group

-**NR¹²R¹⁴**

is a nitrogen-containing heterocycle bound over the nitrogen atom, the nitrogen-containing heterocycle being selected from the group consisting of azetidine, pyrrolidine, piperidine, (1H)tetrahydropyridine, hexahydroazepine, (1H)tetrahydroazepine, octahydroazocine, pyrazolidine, piperazine, hexahydrodiazepine, morpholine, hexahydrooxazepine, thiomorpholine, thiomorpholine-1,1 -dioxide, 5-aza-bicyclo[2.1.1]hexane, 2-aza-bicyclo[2.2.1]heptane, 7-aza-bicyclo[2.2.1]heptane, 2,5-diaza-bicyclo[2.2.1]-heptane, 2-aza-bicyclo[2.2.2]octane, 8-aza-bicyclo[3.2.1]octane, 2,5-diazabicyclo[2.2.2]octane, 9-azabicyclo[3.3.1]nonane, indoline, isoindoline, (1H)-dihydroquinoline, (1H)-tetrahydroquinoline, (2H)-tetrahydroisoquinoline, (1H)-tetrahydroquinoxaline, (4H)-dihydrobenzoxazine, (4H)-dihydrobenzothiazine, (1H)-tetrahydrobenzo[b]azepine, (1H)-tetrahydrobenzo[c]azepine, (1H)-tetrahydrobenzo[d]azepine. (5H)-tetrahydrobenzo[b]oxazepine, (5H)-tet-rahydrobenzo[b]thiazepine, 1,2,3,4-tetrahydro-9H-pyrido[3,4-b]indole, (10H)-dihydroacridine, 1,2,3,4-tetrahydroacridanone, (10H)-phenoxazine, (10H)-phenothiazine, (5H)-dibenzazepine, (5H)-dihydrodibenzazepine, (5H)-octahydrodibenzazepine, (5H)-dihydrodibenzodiazepine, (11H)-dihydrodibenzo[b,e]oxazepine, (11 H)-dihydrodibenzo[b,e]thiazepine, (10H)-dihydrodibenzo[b,f] oxazepine, (10H)-dihydrodibenzo[b,f]thiazepine, and (5H)-tetrahydrodibenzazocine,
- **G3**: is the residue

-**SO₂-(CH₂)ᵣR¹²** (G3),
- **G4**: is the residue 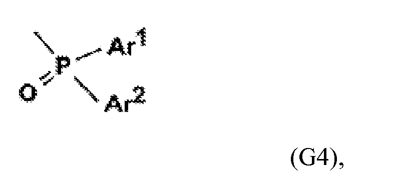 wherein
- **Ar¹** and **Ar²**: are selected independently of each other from phenyl, pyridyl, and naphthyl,
- **G5**: is the residue

-**COR¹⁵** (G5)

wherein
- **R¹⁵**: is selected from trifluoromethyl, C₁-C₆-alkoxy, C₃-C₆-alkenyloxy, and benzyloxy,
wherein aromatic ring systems optionally are substituted independently of each other by one to three substituents independently selected from the group consisting of halogen, cyano, C₁-C₆-alkyl, trifluoromethyl, C₃-C₈-cycloalkyl, phenyl, benzyl, hydroxy, C₁-C₆-alkoxy, C₁-C₆-alkoxy entirely or partially substituted by fluorine; benzyloxy,
phenoxy, mercapto, C₁-C₆-alkylthio, carboxy, C₁-C₆-alkoxycarbonyl, benzyloxycarbonyl, nitro, amino, mono-C₁-C₆-alkylamino, and di-(C₁-C₆-alkyl)-amino,
wherein two adjacent groups in the ring or ring system optionally form an additional ring over a methylenedioxy bridge.

In a further preferred embodiment, the inhibitor is a compound of formula (I), wherein:
- **R¹**: is selected from hydrogen, halogen, cyano, methyl, trifluoromethyl, hydroxy, methoxy and methoxycarbonyl,
- **R²**: is hydrogen or halogen,
- **R³**: is hydrogen,
- **R⁴**: is selected from hydrogen, C₁-C₃-alkyl and hydroxy,
- **k**: is 0 or 1,
- **A**: is selected from C₂-C₆-alkenylene, optionally substituted once or twice by hydroxy or fluorine, or C₄-C₆-alkadienylene, optionally substituted by one or two fluorine atoms, and 1,3,5-hexatrienylene
- **D**: is selected from C₂-C₈-alkylene, optionally substituted by methyl or hydroxy C₂-C₈-alkenylene, optionally substituted by methyl or hydroxy, wherein the double bond optionally is to ring E, and the group consisting of C₂-C₈-alkylene and C₂-C₈-alkenylene, wherein one to three methylene units are isosterically replaced by O, NH, N(CH₃), N(COCH₃), N(SO₂CH₃) or CO,
- **E**: is selected from the residues 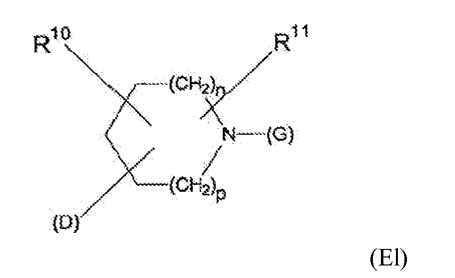 and 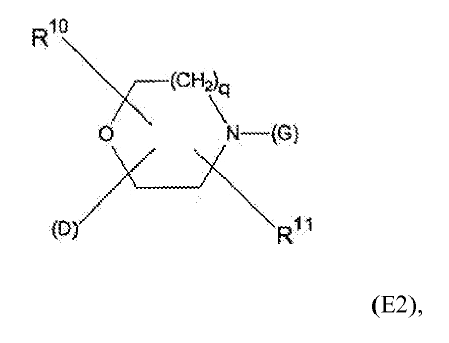 wherein the heterocyclic ring optionally has a double bond and
- **n** and **p**: are, independent of each other, 0, 1, 2 or 3, with the proviso that **n** + **p** ≤ 3
- **q**: is 1 or 2,
- **R¹⁰**: is selected from hydrogen, methyl and hydroxyl,
- **R¹¹**: is hydrogen or an oxo group adjacent to the nitrogen atom,
- **G**: is selected from hydrogen, C₃-C₈-cycloalkyl, methoxycarbonyl, tert- butoxycarbonyl, benzyloxycarbonyl, trifluoroacetyl, diphenylphosphinoyl and the residues

**-(CH₂)ᵣ-(CR¹³R¹⁴)ₛ-R¹²** (G1),

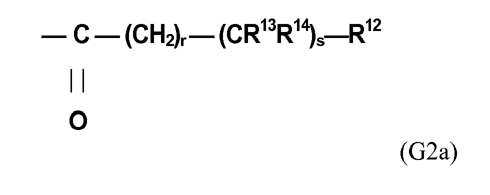

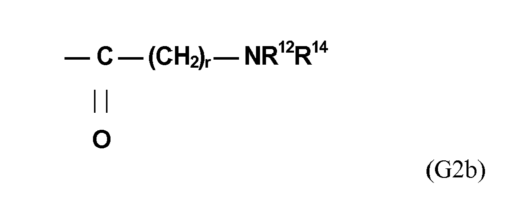

and

-**SO₂-CH₂)ᵣR¹²** (G3)

wherein
- **r**: is 0, 1 or 2,
- **s**: is 0 or 1,
- **R¹²**: is selected from hydrogen, methyl, benzyl, phenyl. the group consisting of indanyl, indenyl, oxoindanyl, naphthyl, dihydronaphthyl, tetrahydronaphthyl, oxotetrahydronaphthyl, flourenyl, oxofluorenyl, anthryl, dihydroanthryl, oxodihydroanthryl, dioxodihydroanthryl, dibenzocycloheptenyl, and oxodibenzocycloheptenyl, dihydrodibenzocycloheptenyl, oxodihydrodibenzocycloheptenyl bound directly or over a methylene group, and the group consisting of furyl, thienyl, pyrrolyl, oxazolyl, isoxazolyl, thiazolyl, isothiazolyl, pyrazolyl, imidazolyl, oxadiazolyl, thiadiazolyl, triazolyl, pyridyl, pyrazinyl, pyridazinyl, pyrimidinyl, imidazothiazolyl, benzofuryl, dihydrobenzofuryl, benzothienyl, dihydrobenzothienyl, indolyl, indolinyl, oxoindolinyl, dioxoindolinyl, benzoxazolyl, oxobenzoxazolinyl, benzisoxazolyl, oxobenzisoxazolinyl, benzothiazolyl, oxobenzthiazolinyl, benzoisothiazolyl, oxobenzoisothiazolinyl, benzimidazolyl, oxobenzimidazolinyl, benzofurazanyl, benzothiadiazolyl, benzotriazolyl, oxazolopyridyl, oxodihydrooxazolopyridyl, thiazolopyridyl, oxodihydrothiazolopyridyl, isothiazolopyridyl, imidazopyridyl, oxodihydroimidazopyridyl, pyrazolopyridyl, thienopyrimidinyl, chromanyl, chromanonyl, benzopyranyl, chromonyl, quinolyl, isoquinolyl, dihydroquinolyl, oxodihydroquinolinyl, tetrahydroquinolyl, oxotetrahydroquinolinyl, benzodioxanyl, quinoxalinyl, quinazolinyl, naphthyridinyl, carbazolyl, tetrahydrocarbazolyl, oxotetrahydrocarbazolyl, pyridoindolyl, acridinyl, oxodihydroacridinyl, phenothiazinyl, dihydrodibenzoxepinyl, benzocycloheptathienyl, oxobenzocycloheptathienyl, dihydrothienobenzothiepinyl, oxodihydrothienobenzothiepinyl, dihydrodibenzothiepinyl, oxodihydrodibenzothiepinyl, dihydrodibenzazepinyl, oxodihydrodibenzazepinyl, octahydrodibenzazepinyl, benzocycloheptapyridyl, oxobenzocycloheptapyridyl, dihydropyridobenzoxepinyl, dihydrodibenzothiazepinyl, and oxodihydrodibenzothiazepinyl, bound directly or over a methylene group,
- **R¹³**: is selected from hydrogen, methyl, benzyl and phenyl,
- **R¹⁴**: is selected from hydrogen, hydroxy, methyl, benzyl, phenyl, and the group consisting of naphthyl, furyl, thienyl, oxazolyl, thiazolyl, pyrazolyl, imidazolyl, oxadiazolyl, thiadiazolyl, pyridyl, benzofuryl, benzothienyl, indolyl, indolinyl, benzoxazolyl, benzothiazolyl, benzim- idazolyl, chromanyl, quinolyl and tetrahydroquinolyl, bound directly or over a methylene group, wherein in formula 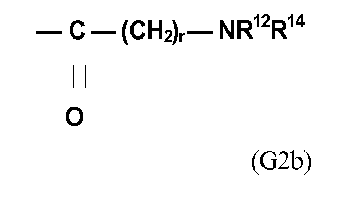 -NR¹²R¹⁴ optionally is selected from pyrrolidine, piperidine, (1H)-tetrahydropyridine, hexahydroazepine, octahydroazocine, piperazine, hexahydrodiazepine, morpholine, hexahydrooxazepine, 2-azabicyclo[2.2.1]heptane, 7-azabicyclo[2.2.1]heptane, 2,5-diazabicyclo[2.2.1]heptane, 8-azabicyclo [3.2.1]octane, 2,5-diazabicyclo[2.2.2]octane, indoline, isoindoline, (1H)-dihydroquinoline, (1H)-tetrahydroqumoline, (2H)-tetrahydroisoquinoline, (1H)-tetrahydroquinoxaline, (4H)-dihydrobenzoxazine, (4H)-dihydrobenzothiazine, (1H)-tetrahydrobenzo[b]azepine, (1H)-tetrahydrobenzo[d]azepine, (5H)-tetrahydrobenzo[b]oxazepine, (5H)-tetrahydrobenzo[b]thiazepine, 1,2,3,4-tetrahydro-9H-pyrido[3,4-b] indol, (10H)-dihydroacridine, 1,2,3,4-tetrahydroacridanone, (5H)-dihydrodibenzazepine, (5H)-dihydrodibenzodiazepine, (11H)-dihydrodibenzo[b,e]oxazepine, (11H)-dihydrodibenzo[b,e]thiazepine, (10H)-dihydrodibenzo[b,f]oxazepine and (5H)-tetrahydrodibenzazocine

In an even further preferred embodiment according to the invention, the inhibitor is a compound of formula (I), wherein:
- **R¹**: is selected from hydrogen, fluorine, chlorine, bromine, methyl, trifluoromethyl and hydroxy,
- **R²**: and
- **R³**: are hydrogen,
- **R⁴**: is hydrogen or hydroxy,
- **k**: is 0 or 1,
- **A**: is C₂-C₄-alkenylene, which is optionally substituted by fluorine,
- **D**: is selected from C₂-C₆-alkylene, C₂-C₆-alkenylene, wherein the double bond optionally is to ring E, and the group consisting of C₂-C₆-alkylene and C₂- C₆-alkenylene, wherein a methylene unit is isosterically replaced by O, NH, N(CH₃) or CO, or an ethylene group is isosterically replaced by NH-CO or CO-NH, or a propylene group is isosterically replaced by NH-CO-O or O-CO-NH,
- **E**: is selected from pyrrolidine, piperidine, 1,2,5,6-tetrahydropyridine, hexahydroazepine, morpholine and hexahydro-1,4-oxazepine, wherein the heterocyclic ring optionally is substituted by an oxo group adjacent to the nitrogen atom,
- **G**: is selected from hydrogen, tert-butoxycarbonyl, diphenylphosphinoyl, and one of the residues

**-(CH₂)ᵣ-(CR¹³R¹⁴)ₛ-R¹²** (G1),

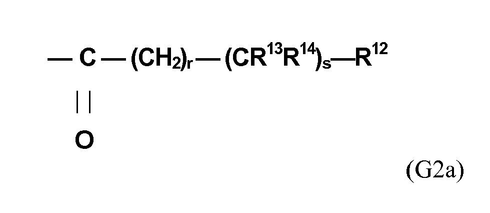

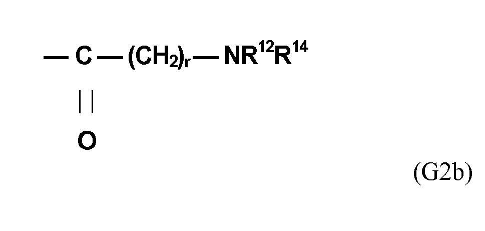

- and: **-SO₂-(CH₂)ᵣR¹²**(G3)
wherein
- **r**: is 0 or 1,
- **s**: is 0 or 1,
- **R¹²**: is selected from hydrogen, methyl, benzyl, phenyl. the group consisting of indenyl, oxoindanyl, naphthyl, tetrahydronaphthyl, flourenyl, oxofluorenyl, anthryl, dihydroanthryl, oxodihydroanthryl, dioxodihydroanthryl and dibenzocycloheptenyl, dihydrodibenzocycloheptenyl, bound directly or over a methylene group, and the group consisting of furyl, thienyl, oxazolyl, thiazolyl, imidazolyl, oxadiazolyl, thiadiazolyl, pyridyl, pyrazinyl, pyrimidinyl, imidazothiazolyl, benzofuryl, benzothienyl, indolyl, oxoindolinyl, dioxoindolinyl, benzoxazolyl, oxobenzoxazolinyl, benzothiazolyl, oxobenzthiazolinyl, benzimidazolyl, oxobenzimidazolinyl, benzofurazanyl, benzotriazolyl, oxazolopyridyl, oxodihydrooxazolopyridyl, thiazolopyridyl, oxodihydrothiazolopyridyl, chromanyl, chromanonyl, benzopyranyl, chromonyl, quinolyl, isoquinolyl, oxodihydroquinolinyl, tetrahydroquinolyl, oxotetrahydroquinolinyl, benzodioxanyl, quinazolinyl, acridinyl, oxodihydroacridinyl, phenothiazinyl, dihydrodibenzoxepinyl, benzocycloheptathienyl, dihydrothienobenzothiepinyl, dihydrodibenzothiepinyl, oxodihydrodibenzothiepinyl, dihydrodibenzazepinyl, oxodihydrodibenzazepinyl, octahydrodibenzazepinyl, benzocycloheptapyridyl. oxobenzocycloheptapyridyl, and dihydrodibenzothiazepinyl, bound directly or over a methylene group,
- **R¹³**: is selected from hydrogen, methyl, benzyl and phenyl,
- **R¹⁴**: is selected from hydrogen, hydroxy, methyl, benzyl, phenyl, and the group consisting of naphthyl, furyl, thienyl, pyridyl, benzofuryl, benzothienyl, indolyl, benzoxazolyl, benzothiazolyl, benzimidazolyl, chromanyl, quinolyl and tetrahydroquinolyl, bound directly or over a methylene group, wherein in formula 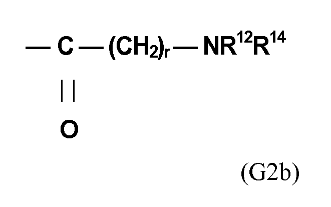 -NR¹²R¹⁴ optionally is selected from pyrrolidine, piperidine, hexahydroazepine, morpholine, 2,5-diazabicyclo[2.2.1]heptane, indoline, isoindoline, (1H)-dihydroquinoline, (1H)-tetrahydroquinoline, (2H)-tetrahydroisoquinoline, (1H)-tetrahydrobenzo[b]azepine, (1H)-tetrahydrobenzo[d]azepine, (5H)-tetrahydrobenzo[b]oxazepine, (5H)-tetrahydrobenzo[b]thiazepine, 1,2,3,4-tetrahydroacridanone, (5H)-dihydrodibenzazepine, (11H)-dihydrodibenzo[b,e]oxazepine, and (11H)-dihydrodibenzo[b,e]thiazepine,
wherein aromatic ring systems optionally are substituted, independently of each other, by one to three substituents which are independently selected from the group consisting of halogen, cyano, C₁-C₆-alkyl, trifluoromethyl, C₃-C₈-cycloalkyl, phenyl, benzyl, hydroxy, C₁-C₆-alkoxy, C₁-C₆-alkoxy which is entirely or partially substituted by fluorine;
benzyloxy, phenoxy, mercapto, C₁-C₆-alkylthio, carboxy, C₁-C₆-alkoxycarbonyl, benzyloxycarbonyl, nitro, amino, mono-C₁-C₆-alkylamino and di-(C₁-C₆-alkyl)-amino, wherein two adjacent groups on the aromatic ring or ring system optionally form an additional ring over a methylenedioxy bridge.

An especially preferred embodiment according to the invention relates to compounds of formula (I), wherein:
- **R¹**: is selected from hydrogen, fluorine, methyl, trifluoromethyl and hydroxy,
- **R²**: and
- **R³**: are hydrogen,
- **R⁴**: is hydrogen or hydroxy,
- **k**: is 0,
- **A**: is ethenylene or 1,3-butadienylene
- **D**: is C₂-C₆-alkylene or C₂-C₆-alkenylene, wherein the double bond optionally is to ring E,
- **E**: is selected from pyrrolidine, piperidine, hexahydroazepine and morpholine,
- **G**: is selected from benzyl, phenethyl, fluorenylmethyl, anthrylmethyl, diphenylmethyl, fluorenyl, dihydrodibenzocycloheptenyl, furylmethyl, thienylmethyl, thiazolylmethyl, pyridylmethyl, benzothienylmethyl, quinolylmethyl, phenyl-thienylmethyl phenyl-pyridylmethyl, dihydrodibenzoxepinyl, dihydrodibenzothiepinyl, acetyl, pivaloyl, phenylacetyl, diphenylacetyl, diphenylpropionyl, naphthylacetyl, benzoyl, naphthoyl, anthrylcarbonyl, oxofluorenylcarbonyl, oxodihydroanthrylcarbonyl, dioxodihydroanthrylcarbonyl, furoyl, pyridylcarbonyl, chromonylcarbonyl, quinolylcarbonyl, naphthylaminocarbonyl, dibenzylaminocarbonyl, benzylphenylaminocarbonyl, diphenylaminocarbonyl, indolinyl-1-carbonyl, dihydrodibenzazepin-N-carbonyl, tetrahydroquinolinyl-N-carbonyl, tetrahy- drobenzo[b]azepinyl-N-carbonyl, methanesulfonyl, phenylsulfonyl, p-toluenesulfonyl, naphthylsulfonyl, quinolinsulfonyl, and diphenylphosphinoyl,
wherein aromatic ring systems optionally are substituted independently of each other by one to three substituents which are independently selected from the group consisting of halogen, cyano, C₁-C₆-alkyl, trifluoromethyl, C₃-C₈-cycloalkyl, phenyl, benzyl, hydroxy, C₁-C₆-alkoxy, C₁-C₆-alkoxy, entirely or partially substituted by fluorine; benzyloxy, phenoxy, mercapto, C₁-C₆-alkylthio, carboxy, C₁-C₆-alkoxycarbonyl, benzyloxycarbonyl, nitro, amino, mono-C1-C6-aₗkylamino and di-(C₁-C₆-alky)-amino, wherein two adjacent groups in the ring or ring system optionally form an additional ring over a methylenedioxy bridge.

A series of exemplary compounds with the respective substituent definitions are listed in the following Table for illustration of the invention.

**Table 1:**

| | | | | | |
|---|---|---|---|---|---|
| Exemplifying compounds of formula (I) according to the invention | | | 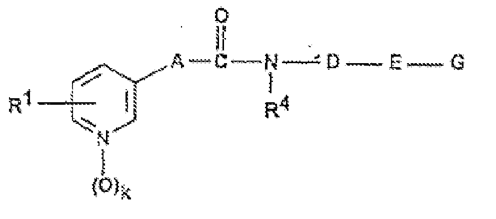 | | |

| Nr | R¹ | k | A | R⁴ | D-E-G |
|---|---|---|---|---|---|
| 1 | H | 0 | CH=CH | H | 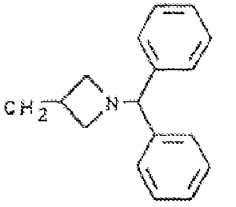 |
| 2 | H | 0 | CH=CH-CH-CH | H | 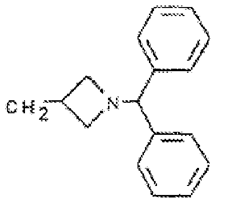 |
| 3 | H | 0 | CH=CH | H | 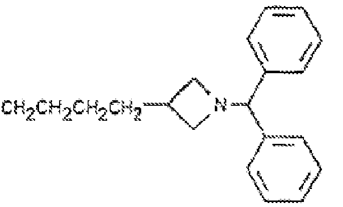 |
| 4 | H | 0 | CH=CH | H | 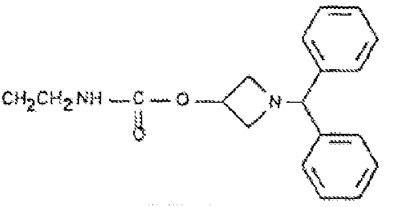 |
| 5 | H | 0 | CH=CH | H | 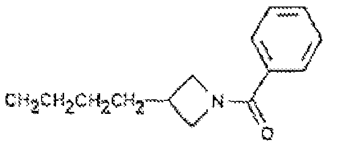 |
| 6 | H | 0 | CH=CH | H | 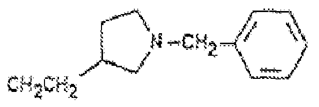 |
| 7 | H | 0 | CH=CH-CH=CH | H | 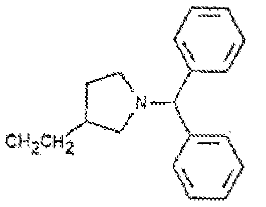 |
| 8 | H | 0 | CH=CH(CH₂)₂ | H | 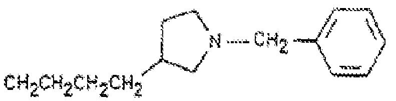 |
| 9 | H | 0 | CH=CH | H | 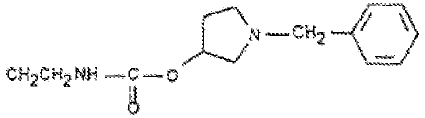 |
| 10 | H | 0 | CH=CH | H | 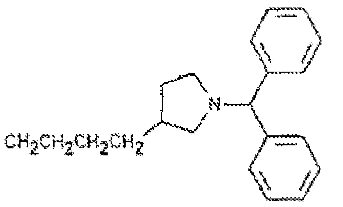 |
| 11 | H | 0 | CH=CH | H | 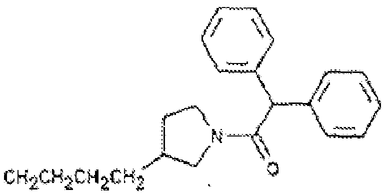 |
| 12 | H | 0 | CH=CH | H | 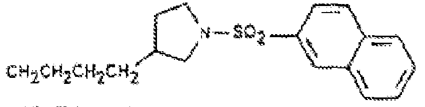 |
| 13 | H | 0 | CH=CH | H | 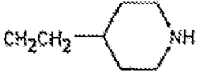 |
| 14 | H | 0 | CH=CH | H | 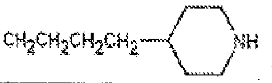 |
| 15 | H | 0 | CH=CH-CH=CH | H | 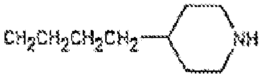 |
| 16 | H | 0 | CH=CH | H | 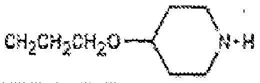 |
| 17 | H | 0 | CH=CH | H | 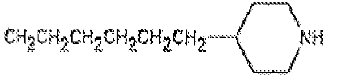 |
| 18 | H | 0 | CH=CH-CH=CH | H | 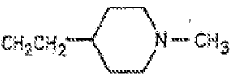 |
| 19 | H | 0 | CH=CH-CH=CH | H | 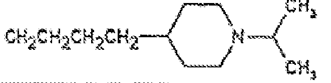 |
| 20 | H | 0 | CH=(CH₂)₂ | H | 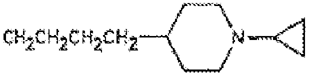 |
| 21 | H | 0 | CH=CH | H | 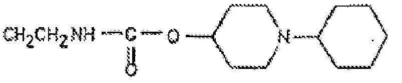 |
| 22 | H | 0 | CH=C CN | H | 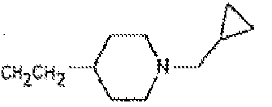 |
| 23 | H | 0 | CH=CH | H | 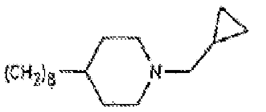 |
| 24 | H | 0 | CH=CH | H | 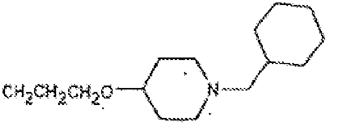 |
| 25 | H | 0 | CH=CH | H | 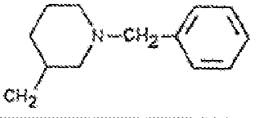 |
| 26 | H | 0 | CH=CH | H | 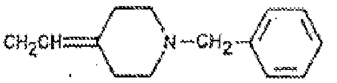 |
| 27 | H | 0 | CH=CH-CH=CH | H | 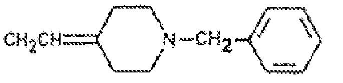 |
| 28 | H | 1 | CH=CH | H | 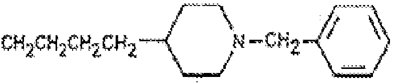 |
| 29 | H | 0 | CH=CH | OH | 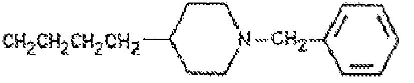 |
| 30 | H | 0 | 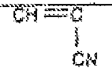 | H | 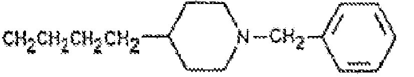 |
| 31 | H | 0 | C≡C | H | 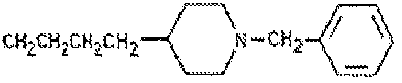 |
| 32 | H | | CH=CH (CH₂)₂ | H | 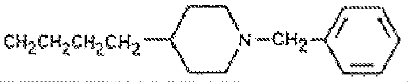 |
| 33 | H | 0 | CH=CH-CH=CH | H | 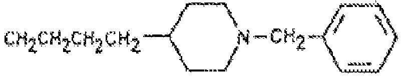 |
| 34 | 2-F | 0 | CH=CH-CH=CH | H | 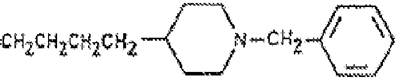 |
| 35 | H | 0 | (CH=CH)₃ | H | 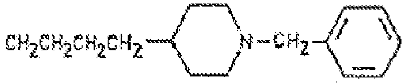 |
| 36 | H | 0 | CH=CH | H | 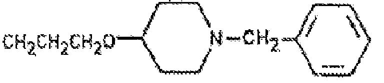 |
| 37 | H | 0 | CH=CH | H | 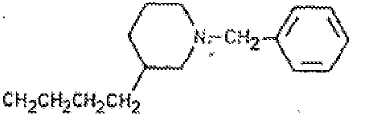 |
| 38 | H | 0 | CH=CH | H | 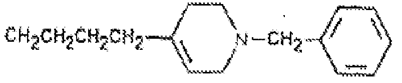 |
| 39 | H | 0 | CH=CH | H | 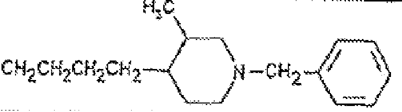 |
| 40 | H | 0 | CH=CH | H | 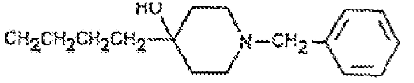 |
| 41 | H | 0 | CH=CH | H | 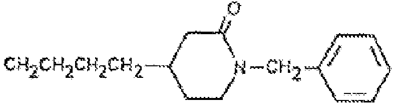 |
| 42 | H | 0 | CH=CH-CH=CH | H | 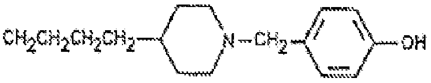 |
| 43 | H | 0 | CH=CH-CH=CH | H | 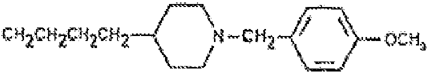 |
| 44 | H | 0 | CH=CH-CH=CH | H | 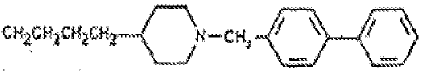 |
| 45 | H | 0 | CH=CH-CH=CH | H | 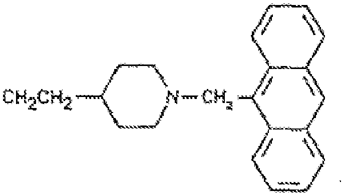 |
| 46 | H | 0 | C≡C | H | 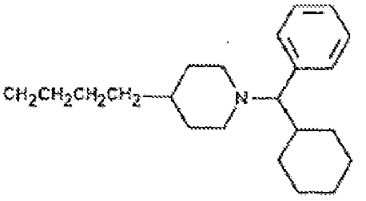 |
| 47 | H | 0 | CH=CH | H | 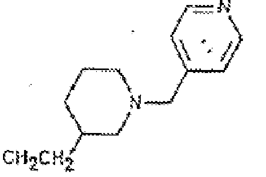 |
| 48 | H | 0 | CH=CH-CH=CH | H | 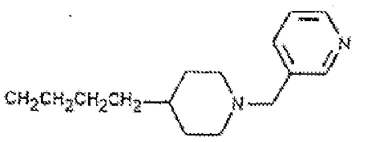 |
| 49 | H | 0 | CH=CH | H | 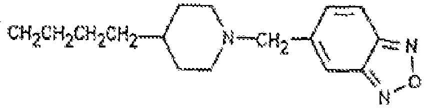 |
| 50 | H | 0 | CH=CH | H | 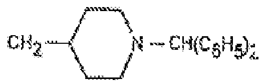 |
| 51 | H | 0 | CH=CH-CH=CH | H | 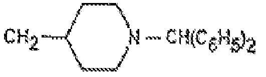 |
| 52 | H | 1 | CH=CH | H | 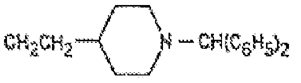 |
| 53 | H | 0 | CH=CH(CH₂)₂ | H | 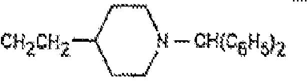 |
| 54 | H | 0 | CH=CHCH₂CHF | H | 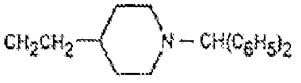 |
| 55 | H | 0 | CH=CH | H | 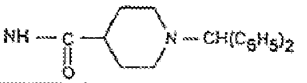 |
| 56 | H | 0 | CH=CH | H | 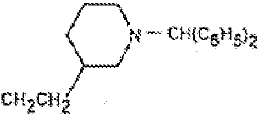 |
| 57 | H | 0 | CH=CH-CH=CH | H | 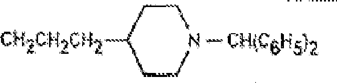 |
| 58 | H | 0 | CH=CH | H | 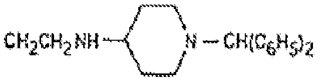 |
| 59 | H | 1 | CH=CH | H | 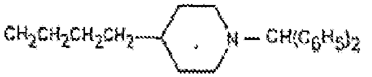 |
| 60 | H | 0 | CH=CH | CH | 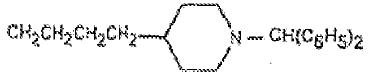 |
| 61 | H | 0 | 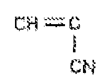 | H | 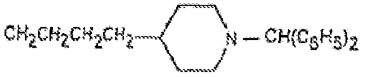 |
| 62 | H | 0 | C=C | H | 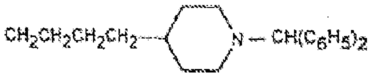 |
| 63 | H | 0 | CH=CH(CH₂)₂ | H | 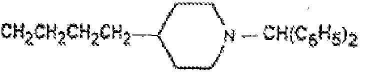 |
| 64 | H | 0 | 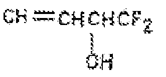 | H | 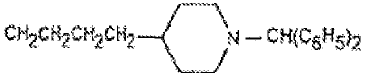 |
| 65 | H | 0 | (CH₂)₂CH=CH | H | 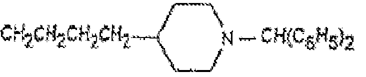 |
| 66 | H | 0 | CH=CH-CH=CH | H | 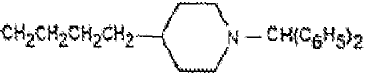 |
| 67 | H | 0 | CH=CH-CH=CH | CH₃ | 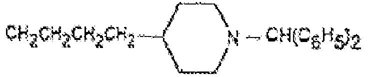 |
| 68 | 2·F | 0 | CH=CH-CH=CH | H | 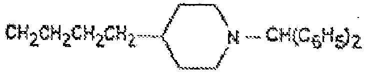 |
| 69 | 2·F | 0 | CH=CH-CH=CH | OH | 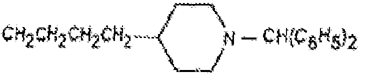 |
| 70 | 4·F | 0 | CH=CH-CH=CH | H | 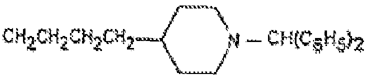 |
| 71 | 5·F | 0 | CH=CH-CH=CH | H | 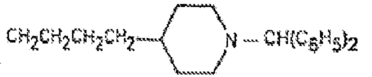 |
| 72 | 6·F | 0 | CH=CH-CH=CH | H | 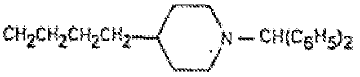 |
| 73 | 2·Cl | 0 | CH=CH-CH=CH | H | 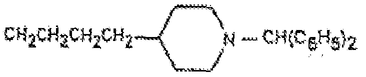 |
| 74 | 6-CH₃ | 0 | CH=CH-CH=CH | H | 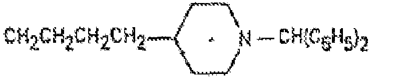 |
| 75 | 2·OH | 0 | CH=CH-CH=CH | H | 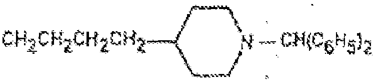 |
| 76 | H | 0 | (CH=CH)₂ | H | 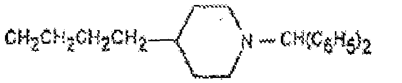 |
| 77 | H | 0 | CH=CH | H | 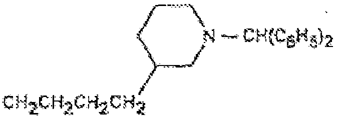 |
| 78 | 2-F | 0 | CH=CH | H | 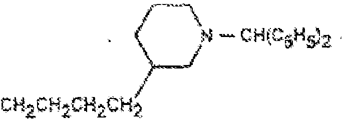 |
| 79 | 5·F | 0 | CH=CH | H | 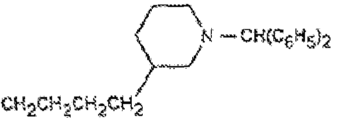 |
| 80 | 6-CH₃O | 0 | CH=CH | H | 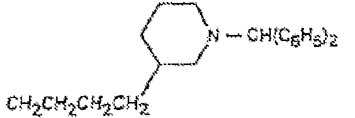 |
| 81 | H | 0 | CH=CH-CH=CH | H | 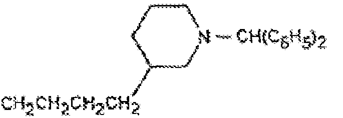 |
| 82 | H | 0 | CH=CH | H | 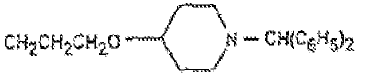 |
| 83 | H | 0 | CH=CH-CH=CH | H | 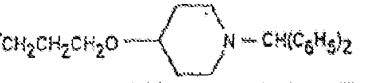 |
| 84 | H | 0 | CH=CH | H | 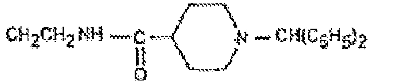 |
| 85 | H | 0 | CH=CH | H | 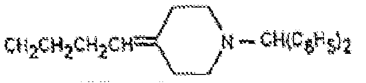 |
| 86 | H | 0 | CH=CH | H | 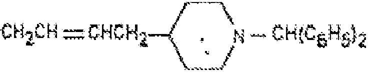 |
| 87 | H | 0 | CH=CH | H | 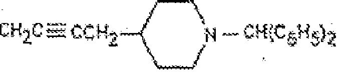 |
| 88 | H | 0 | CH=CH | H | 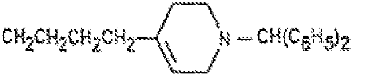 |
| 89 | H | 0 | CH=CH-CH=CH | H | 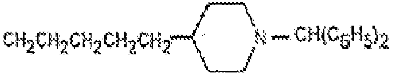 |
| 90 | H | 0 | CH=CH | H | 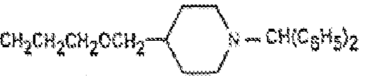 |
| 91 | H | 0 | CH=CH-CH=CH | H | 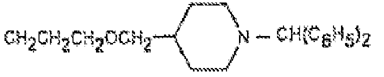 |
| 92 | H | 0 | CH=CH | H | 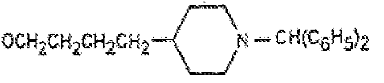 |
| 93 | H | 0 | CH=CH | H | 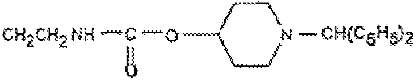 |
| 94 | H | 0 | CH=CH-CH=CH | H | 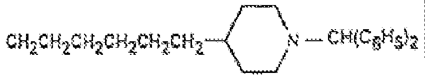 |
| 95 | H | 0 | CH=CH | H | 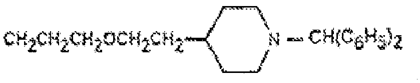 |
| 96 | H | 0 | CH=CH | H | 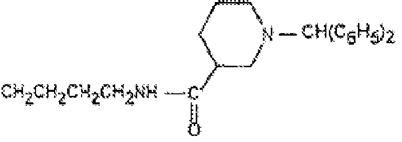 |
| 97 | H | 0 | CH=CH | H | 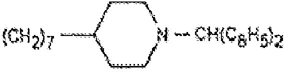 |
| 98 | H | 0 | CH=CH | H | 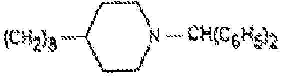 |
| 99 | H | 0 | CH=CH | H | 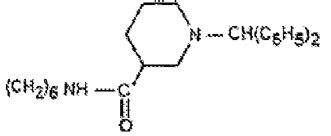 |
| 100 | H | 0 | CH=CH | H | 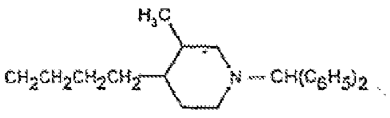 |
| 101 | H | 0 | CH=CH | H | 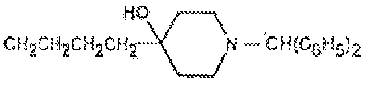 |
| 102 | H | 0 | CH=CH | H | 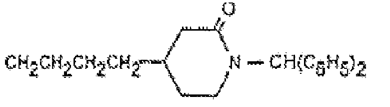 |
| 103 | H | 0 | CH=CH-CH=CH | H | 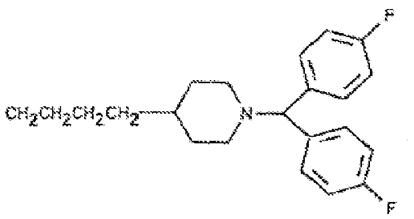 |
| 104 | H | 0 | C≡C | H | 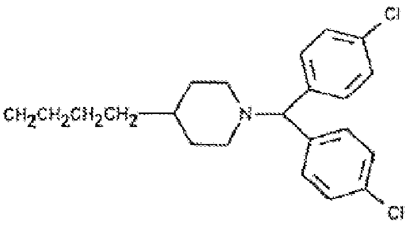 |
| 105 | H | 0 | CH=CH-CH=CH | H | 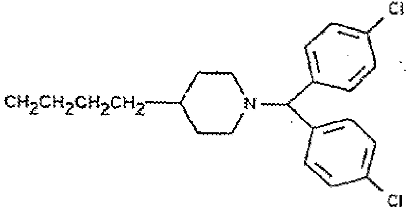 |
| 106 | H | 0 | C≡C | H | 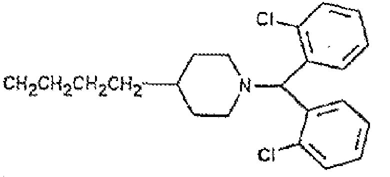 |
| 107 | H | 0 | (CH₂)₂CH=CH | H | 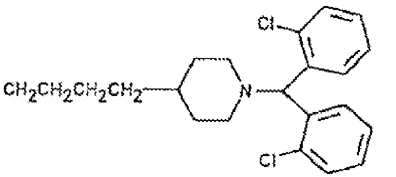 |
| 108 | H | 0 | CH=CH-CH=CH | H | 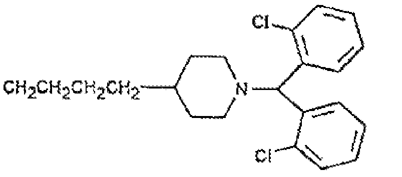 |
| 109 | H | 0 | CH=CH-CH=CH | H | 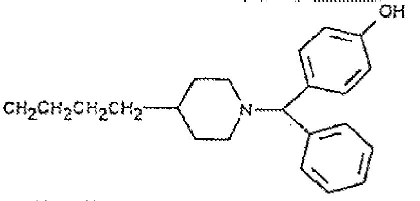 |
| 110 | H | 0 | CH=CH-CH=CH | H | 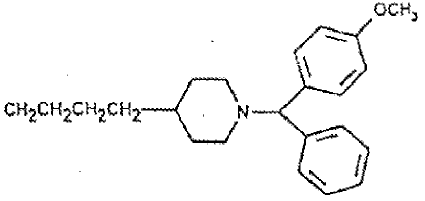 |
| 111 | H | 0 | CH=CH-CH=CH | H | 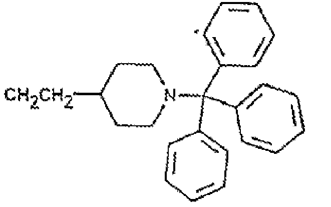 |
| 112 | H | 0 | CH=CH-CH=CH | H | 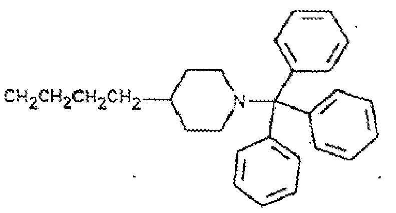 |
| 113 | H | 0 | CH=CH | H | 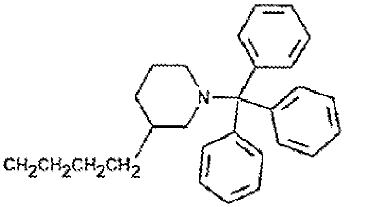 |
| 114 | H | 0 | CH=CH-CH=CH | H | 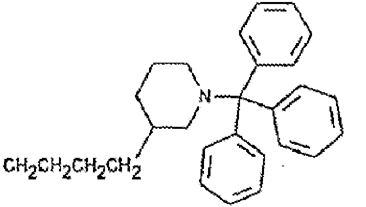 |
| 115 | H | 0 | CH=CH | H | 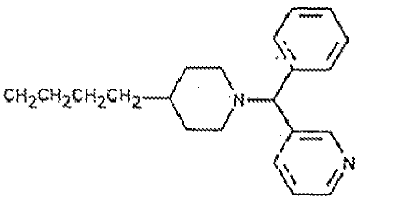 |
| 116 | H | 0 | CH-CH-CH=CH | H | 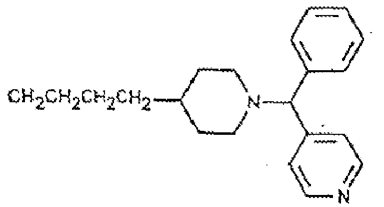 |
| 117 | H | 0 | CH=CH | H | 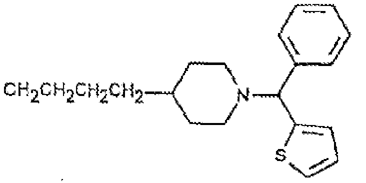 |
| 118 | H | 0 | CH=CH-CH=CH | H | 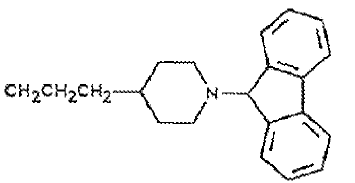 |
| 119 | H | 0 | CH=C CN | H | 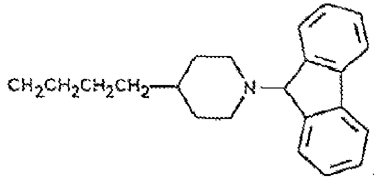 |
| 120 | H | 0 | CH=CH-CH=CH | H | 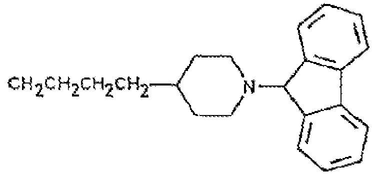 |
| 121 | H | 0 | CH=CHCH₂CC-F | H | 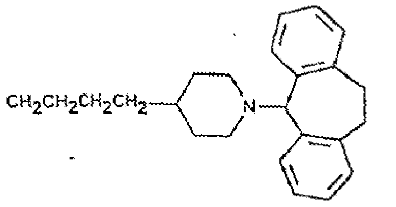 |
| 122 | H | 0 | CH=CH-CH=CH | H | 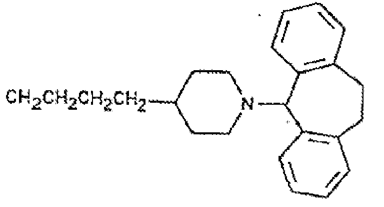 |
| 123 | H | 0 | C≡C | H | 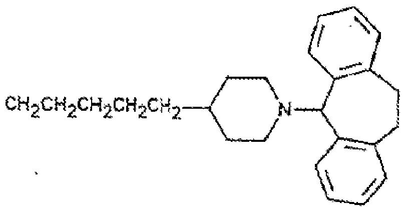 |
| 124 | H | 0 | CH=CH | H | 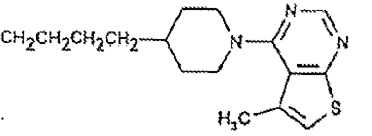 |
| 125 | H | 0 | CH=CH-CH=CH | H | 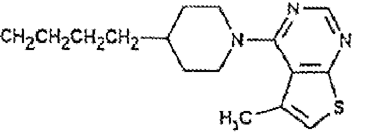 |
| 126 | H | 0 | CH=CH | H | 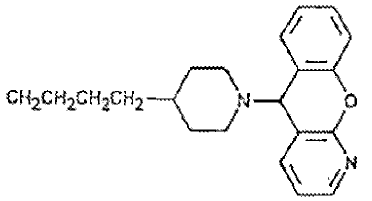 |
| 127 | H | 0 | CH=CH | H | 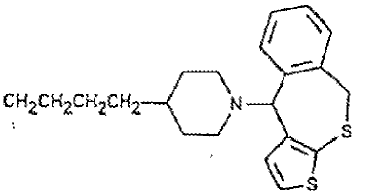 |
| 128 | H | 0 | CH=CH | H | 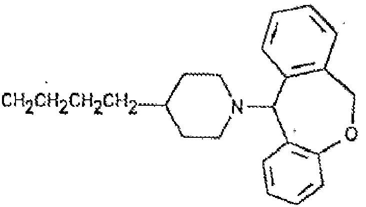 |
| 129 | H | 0 | CH=CH-CH=CH | H | 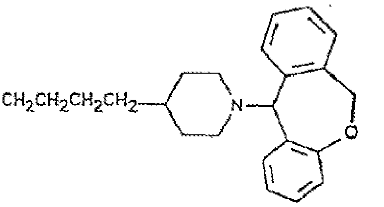 |
| 130 | H | 0 | CH=CH | H | 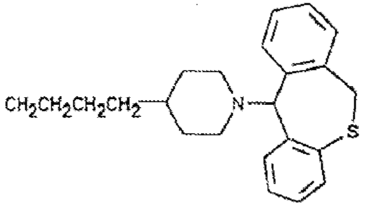 |
| 131 | H | 0 | CH=CH-CH=CH | H | 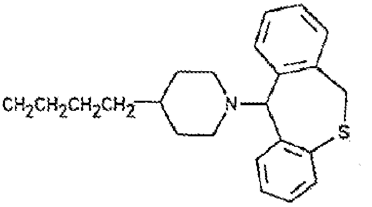 |
| 132 | H | 0 | CH=CH | H | 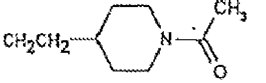 |
| 133 | H | 0 | CH=CH-CH=CH | H | 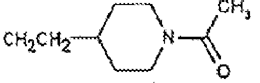 |
| 134 | H | 0 | CH=CH | H | 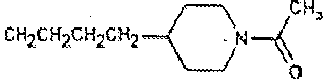 |
| 135 | H | 0 | CH=CH-CH=CH | H | 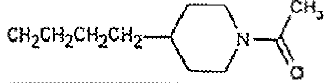 |
| 136 | H | 0 | CH=CH | H | 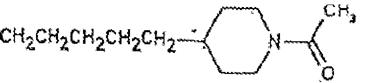 |
| 137 | H | 0 | CH=CH | H | 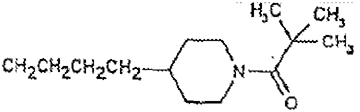 |
| 138 | H | 0 | CH=CH | H | 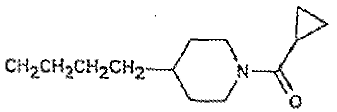 |
| 139 | H | 0 | CH=CH-CH=CH | H | 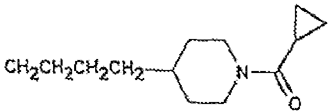 |
| 140 | H | 0 | CH-CH | H | 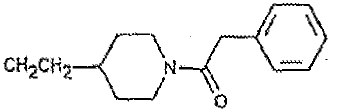 |
| 141 | H | 0 | CH=CH | H | 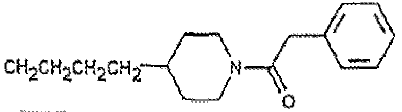 |
| 142 | H | 0 | CH=CH-CH=CH | H | 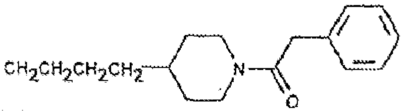 |
| 143 | H | 0 | CH=CH(CH₂)₂ | H | 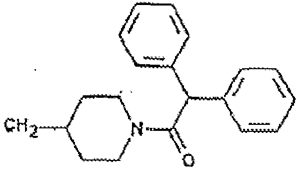 |
| 144 | H | 0 | CH=CH | H | 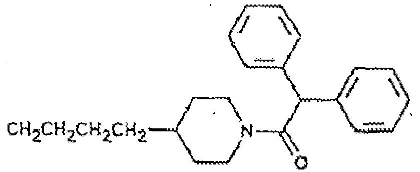 |
| 145 | H | 0 | CH=CH-CH=CH | H | 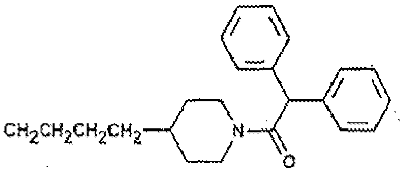 |
| 146 | H | 0 | CH=CH | H | 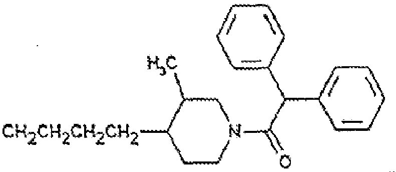 |
| 147 | H | 0 | CH=CH | H | 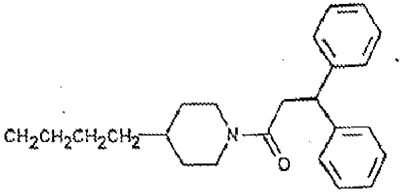 |
| 148 | H | 0 | CH=CH-CH=CH | H | 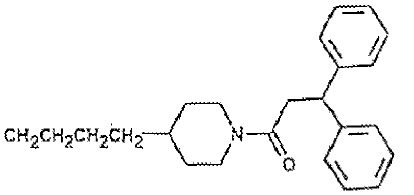 |
| 149 | H | 0 | CH=CH | H | 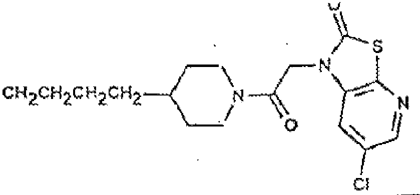 |
| 150 | H | 0 | CH=CH-CH=CH | H | 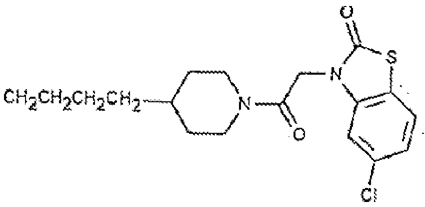 |
| 151 | H | 0 | CH=CH | H | 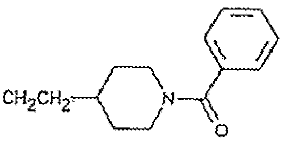 |
| 152 | H | 0 | CH=CH | H | 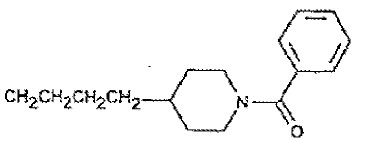 |
| 153 | H | 0 | 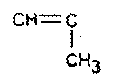 | H | 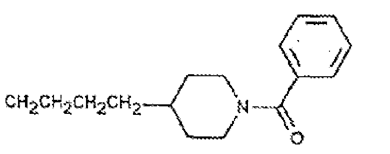 |
| 154 | H | 0 | 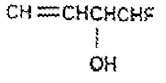 | H | 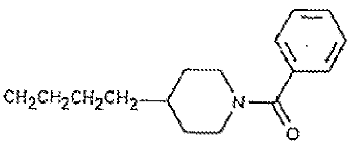 |
| 155 | H | 0 | CH=CH-CH=CH | H | 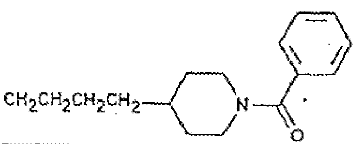 |
| 156 | H | 0 | CH=CH | H | 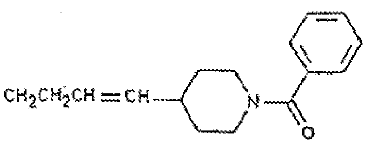 |
| 157 | H | 0 | CH=CH | CH₃ | 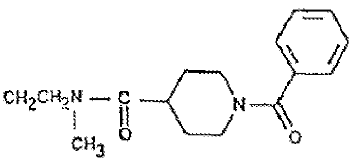 |
| 158 | H | 0 | CH=CH | H | 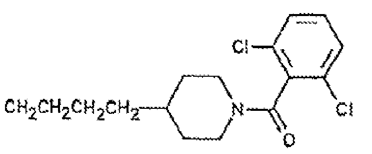 |
| 159 | H | O | CH=CH | H | 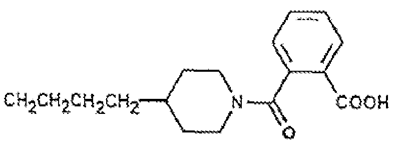 |
| 160 | H | 0 | CH=CH | H | 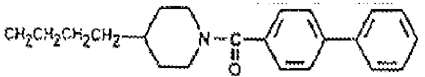 |
| 161 | H | 0 | CH=CH-CH=CR | H | 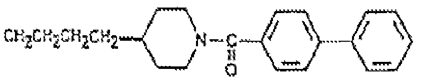 |
| 162 | H | 0 | H=CH | H | 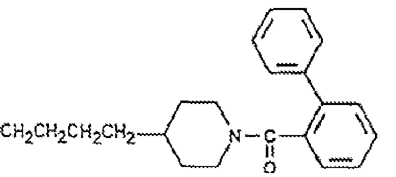 |
| 163 | H | 0 | (CH₂)₂CH=CH | H | 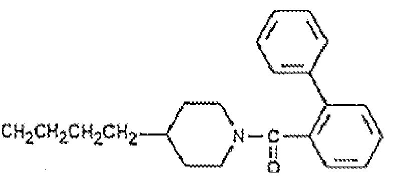 |
| 164 | H | 0 | CH=CH CH=CH | H | 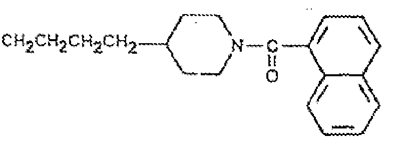 |
| 165 | 2-F | 0 | CH=CH | H | 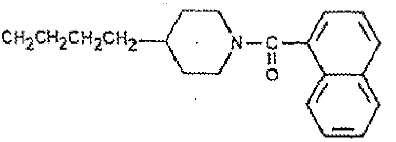 |
| 166 | H | 0 | CH=CH·CH=CH | H | 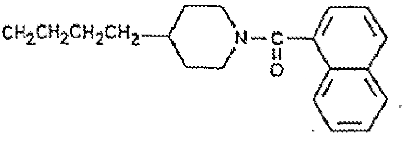 |
| 167 | H | 0 | CH=CH | H | 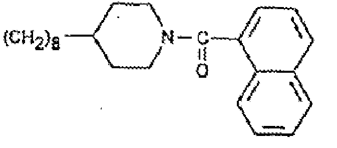 |
| 168 | H | 0 | CH=CH | H | 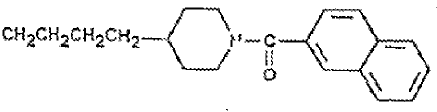 |
| 169 | H | 0 | CH=CH-CH=CH | H | 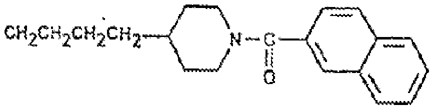 |
| 170 | H | 0 | CH=CH | H | 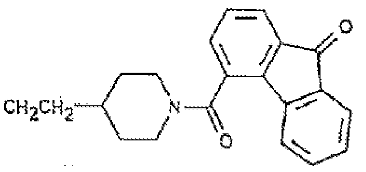 |
| 171 | H | 0 | CH=CH | H | 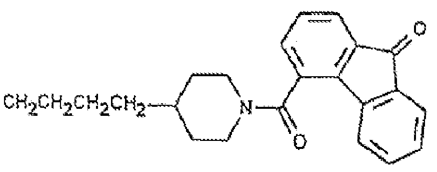 |
| 172 | H | 0 | CH=CH-CH=CH | H | 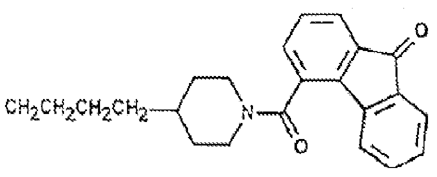 |
| 173 | H | 0 | CH=CH | H | 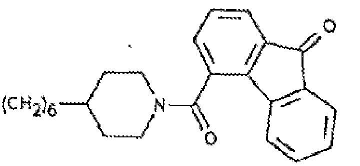 |
| 174 | H | 0 | CH=CH | H | 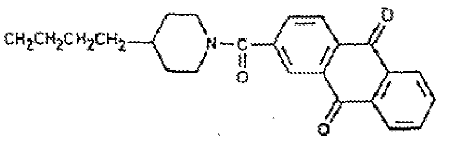 |
| 175 | 4-F | 0 | CH=CH | H | 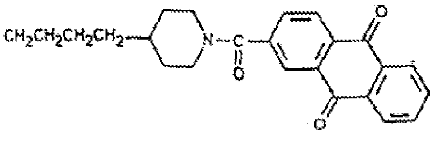 |
| 176 | H | 0 | CH=CH-CH=CH | H | 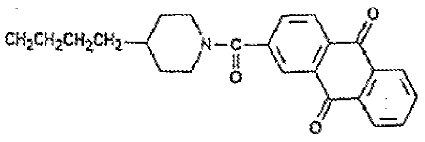 |
| 177 | H | 0 | CH=CH | H | 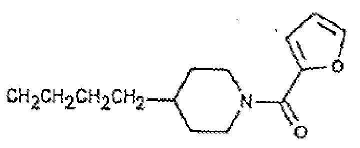 |
| 178 | H | 0 | CH=CH | H | 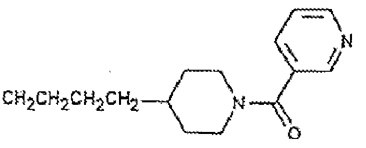 |
| 179 | H | 0 | (CH=CH)₃ | H | 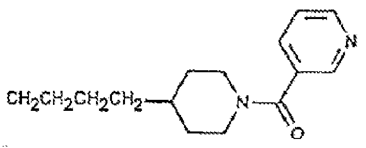 |
| 180 | H | 0 | CH=CH | H | 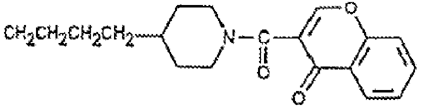 |
| 181 | H | 0 | CH=CH-CH=CH | H | 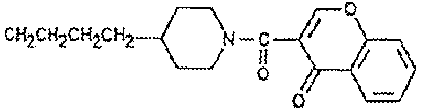 |
| 182 | H | 0 | CH=CH | H | 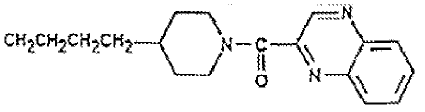 |
| 183 | H | 0 | C≡C | H | 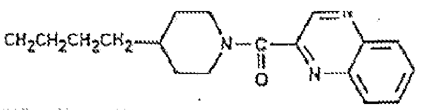 |
| 184 | H | 0 | CH=CH | H | 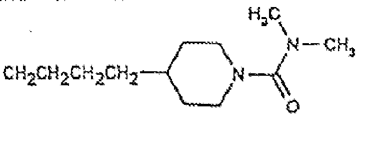 |
| 185 | H | 0 | CH=CH | H | 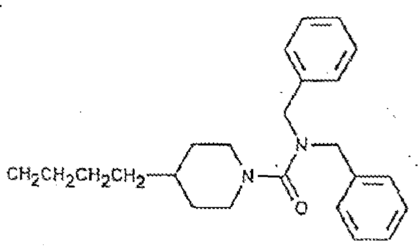 |
| 186 | H | 0 | CH=CH-CH=CH | H | 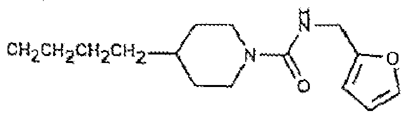 |
| 187 | H | 0 | CH=CH | H | 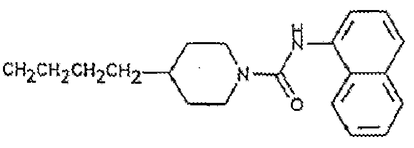 |
| 188 | 2-Cl | 0 | CH=CH | H | 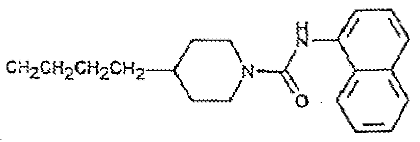 |
| 189 | H | 0 | CH=CH | H | 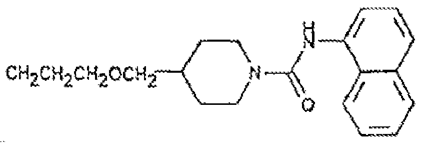 |
| 190 | H | 0 | CH=CH | H | 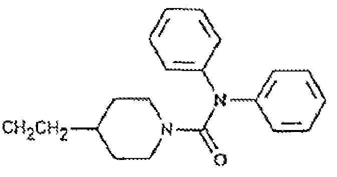 |
| 191 | H | 0 | CH=CH | H | 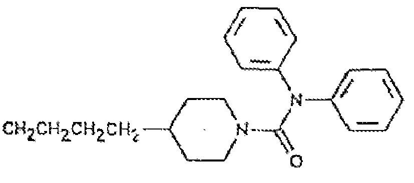 |
| 192 | H | 0 | CH=CH-CH=CH | H | 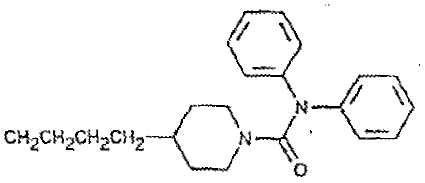 |
| 193 | H | 0 | CH=CH | H | 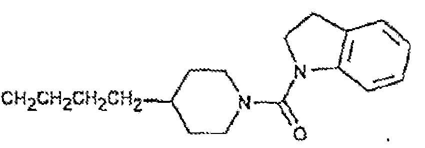 |
| 194 | H | 0 | CH=CH-CH=CH | H | 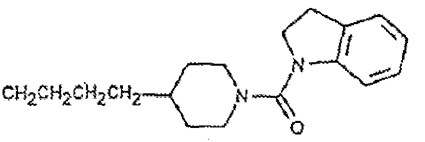 |
| 195 | H | 0 | CH=CH | H | 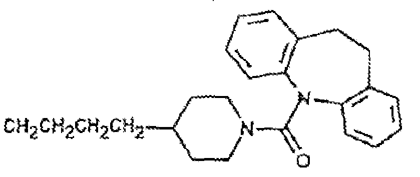 |
| 196 | H | 0 | CH=CH-CH=CH | H | 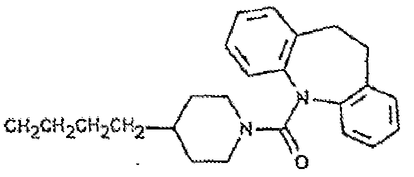 |
| 197 | H | 0 | CH=CH | H | 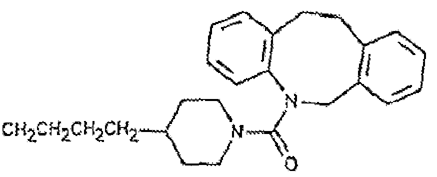 |
| 198 | H | 0 | CH=CH-CH=CH | H | 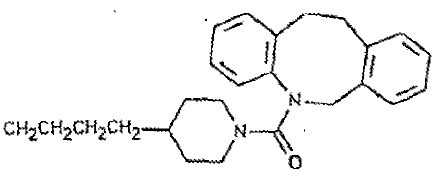 |
| 199 | H | 0 | CH=CH | H | 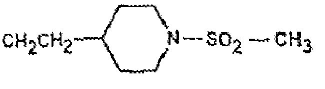 |
| 200 | H | 0 | CH=CH | H | 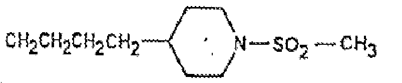 |
| 201 | H | 0 | CH=CH-CH=CH | H | 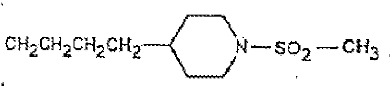 |
| 202 | H | 0 | CH=CH | H | 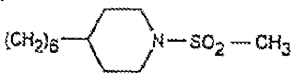 |
| 203 | H | 0 | CH=CH | H | 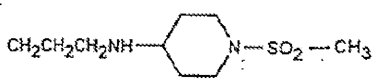 |
| 204 | H | 0 | CH=CH | H | 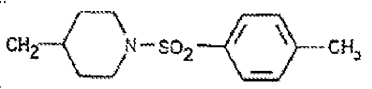 |
| 205 | H | 0 | CH=CF | H | 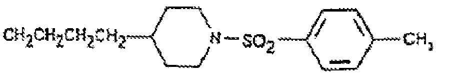 |
| 206 | H | 0 | CH=CH-CH=CH | H | 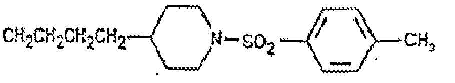 |
| 207 | H | 0 | CH=CH | H | 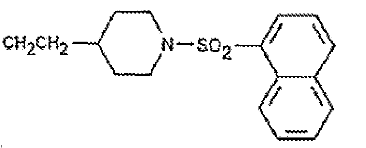 |
| 208 | H | 0 | CH=CH | H | 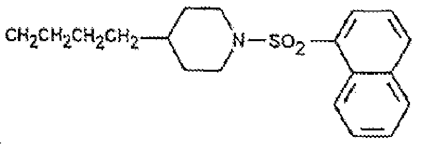 |
| 209 | H | 0 | CH=CH | H | 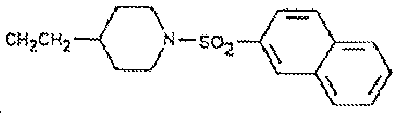 |
| 210 | H | 0 | CH=CH-CH=CH | H | 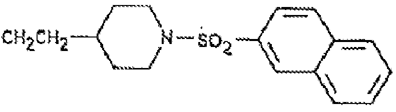 |
| 211 | H | 0 | CH=CH | H | 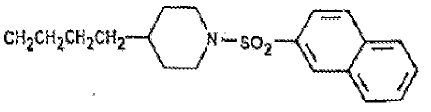 |
| 212 | H | 0 | C≡C | H | 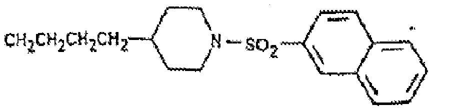 |
| 213 | H | 0 | (CH₂)₂CH=CH | H | 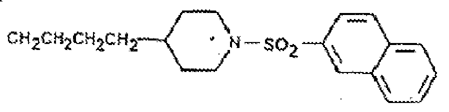 |
| 214 | H | 0 | CH=CH-CH=CH | H | 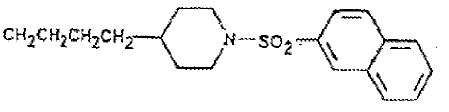 |
| 215 | H | 0 | CH=CH | H | 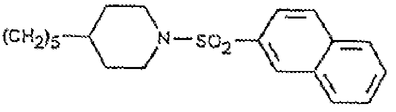 |
| 216 | H | 0 | CH=CH | H | 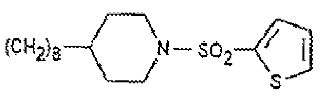 |
| 217 | H | 0 | CH=CH | H | 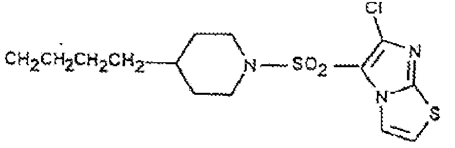 |
| 218 | H | 0 | CH=CH-CH=CH | H | 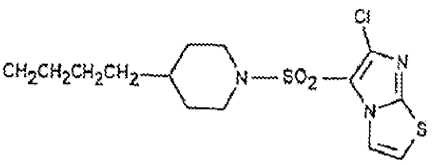 |
| 219 | H | 0 | CH=CH | H | 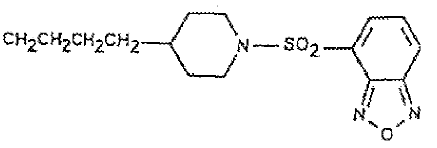 |
| 220 | H | 0 | CH=CH | H | 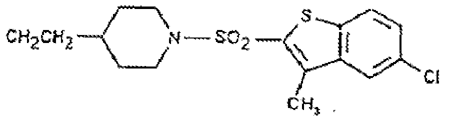 |
| 221 | H | 0 | CH=CH | H | 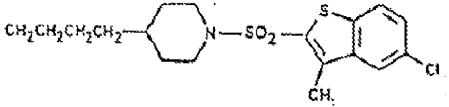 |
| 222 | H | 0 | CH=CH-CH=CH | H | 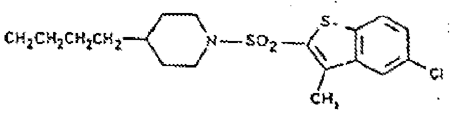 |
| 223 | H | 0 | CH=CH | H | 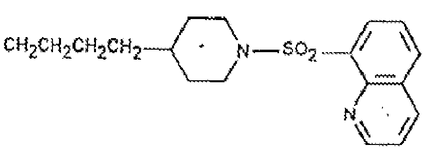 |
| 224 | H | 0 | CH=CH | H | 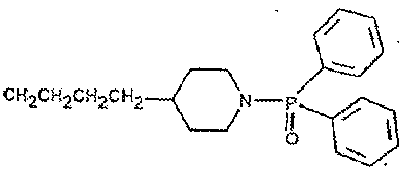 |
| 225 | H | 0 | CH=CH-CH=CH | H | 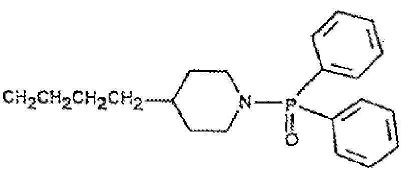 |
| 226 | H | 0 | CH=CH | H | 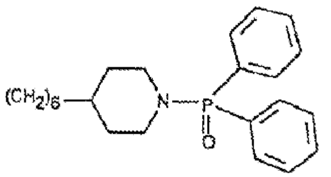 |
| 227 | H | | 0 CH=CH | H | 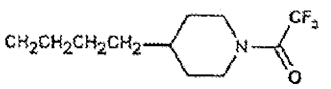 |
| 228 | H | 0 | CH=CH-CH=CH | H | 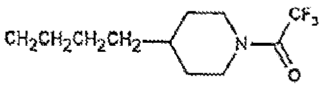 |
| 229 | H | 0 | CH=CH | H | 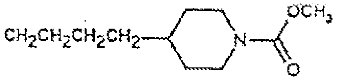 |
| 230 | H | 0 | CH=CH-CH=CH | H | 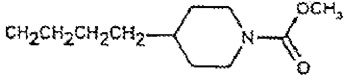 |
| 231 | H | 0 | CH=CH | H | 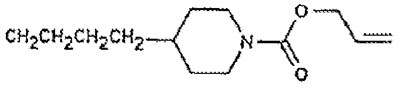 |
| 232 | H | 0 | CH=CH | H | 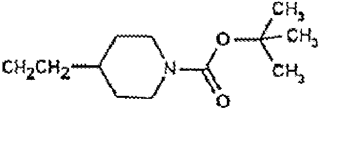 |
| 233 | H | 0 | CH=CH-CH=CH | H | 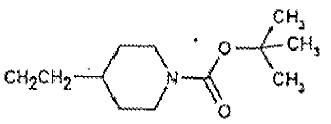 |
| 234 | H | 0 | CH=CH | H | 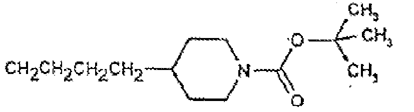 |
| 235 | H | 0 | CH=CH-CH=CH | H | 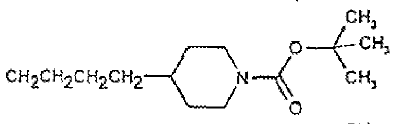 |
| 236 | H | 0 | C≡C | H | 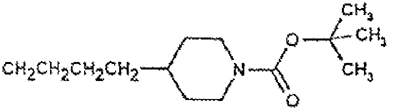 |
| 237 | H | 0 | (CH=CH)₃ | H | 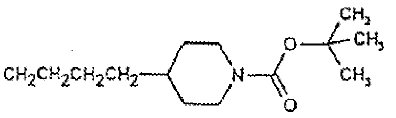 |
| 238 | H | 0 | CH=CH | H | 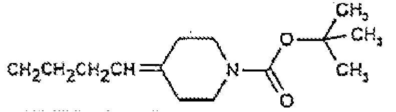 |
| 239 | H | 0 | CH=CH | H | 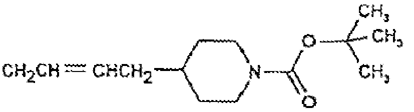 |
| 240 | H | 0 | CH=CH | H | 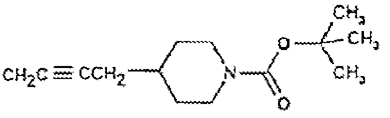 |
| 241 | H | 0 | CH=CH | H | 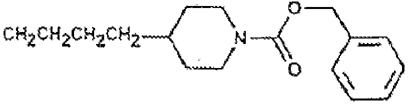 |
| 242 | H | 0 | CH=CH | H | 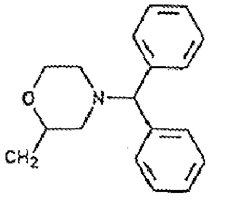 |
| 243 | H | 0 | CH=CH-CH=CH | H | 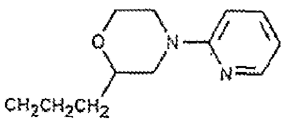 |
| 244 | H | 0 | CH=CH | H | 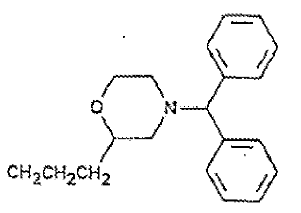 |
| 245 | H | 0 | CH=CH | H | 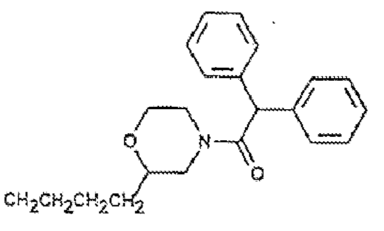 |
| 246 | H | 0 | CH=CH | H | 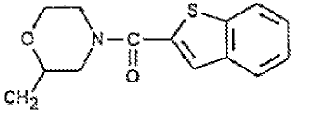 |
| 247 | H | 0 | CH=CH-CH=HC | H | 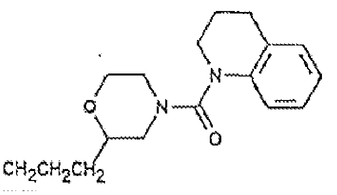 |
| 248 | H | 0 | CH=CH | H | 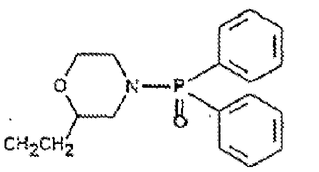 |
| 249 | H | 0 | CH=CH-CH=CH | H | 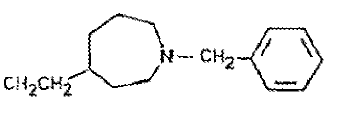 |
| 250 | H | 0 | CH=CH | H | 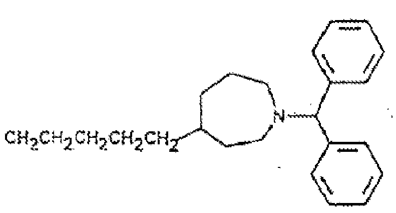 |
| 251 | H | 0 | CH=CH | H | 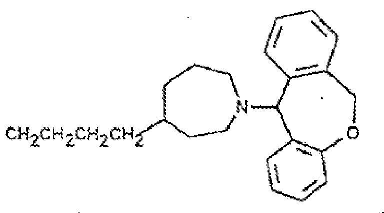 |
| 252 | H | 0 | CH=CH | H | 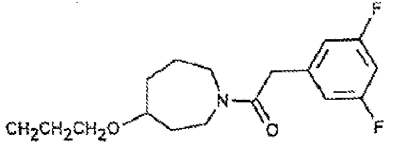 |
| 253 | H | 0 | CH=CH-CH=CH | H | 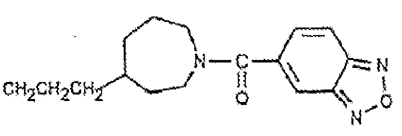 |
| 254 | H | 0 | C≡C | H | 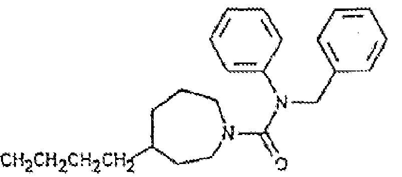 |
| 255 | H | 0 | CH=CH | H | 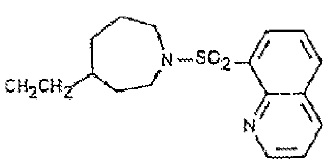 |
| 256 | H | 0 | CH=CH | H | 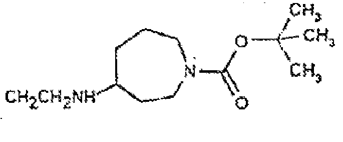 |
| 257 | H | 0 | CH=CH | H | 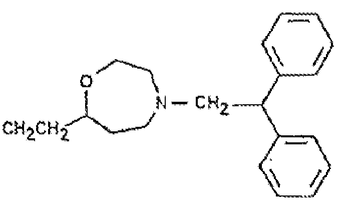 |
| 258 | H | 0 | CH=CH | H | 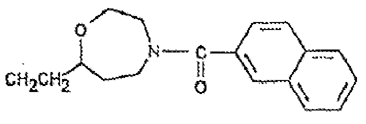 |
| 259 | H | 0 | CH=CH | H | 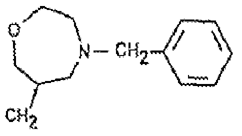 |
| 260 | H | 0 | CH=CH-CH=CH | H | 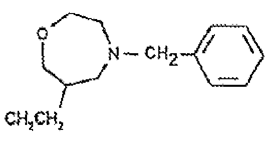 |

More preferably, the inhibitor is (E)-N[4-(1-benzoylpiperidin-4-yl) butyl]-3-pyridin-3-yl)-acrylamide.

Synthesis methods are for example described in EP0923570.

In an aspect of the above described embodiments of the invention the ischemia-reperfusion injury is selected from; intestinal ischemia reperfusion injury, renal ischemia reperfusion injury, hepatic ischemia reperfusion injury, ischemia-reperfusion injury of other internal organs such as the lungs or heart, central nervous system ischemia-reperfusion injury, ischemia reperfusion injury of the limbs or digits, or ischemia reperfusion injury of any transplanted organ. In one aspect, the ischemia reperfusion injury is associated with an autoimmune disease. In one aspect the ischemia reperfusion injury is associated with a disease or condition selected from stroke, traumatic brain injury, spinal cord injury, trauma induced hypovolemic shock and rheumatoid arthritis.

This invention will be better understood from the Experimental Details that follow. However, one skilled in the art will readily appreciate that the specific methods and results discussed are merely illustrative of the invention as described more fully in the claims which follow thereafter, and are not to be considered in any way limited thereto.

### Figures

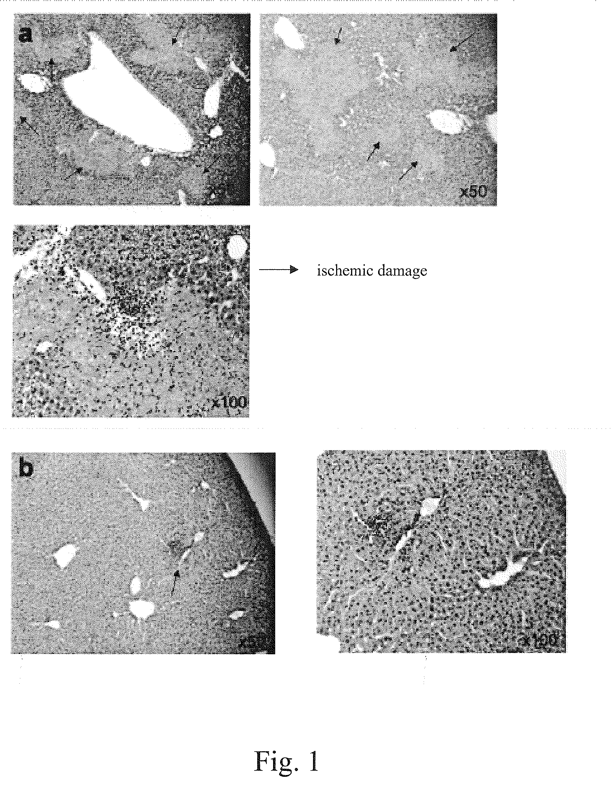

Figure 1: Mice were left untreated (Figure 1a) or injected with FK866 (10mg/kg, Figure 1b) 3 hours before undergoing warm partial hepatic ischemia reperfusion. Livers were removed 24 hours after surgery and analyzed for necrosis /inflammation.
Figure 1a: Control mice displayed typical necrosis (two top panels) and mononuclear cell infiltration (bottom panel).
Figure 1b: Mice treated with FK866 displayed reduced necrosis and rare, scattered mononuclear cell infiltration.

### Example

Ischemia/reperfusion (I/R) injury is a major obstacle affecting graft function after liver transplantation. Although the cause of this injury is multifactorial, increasing experimental evidence suggests an important role for Kupffer cells in initiating the inflammatory cascade leading to pathogenesis. TNFα is though to play an important role during I/R (see Farmer DG et al, Transplantation Reviews 2000 14:106). To evaluate the potential protective role of FK866 on hepatic ischemia/reperfusion injury, two groups of mice were injected with FK866 (10 mg/kg) or solvent 3 hours before performing a partial warm liver ischemia. The left liver lobe of female C57BL/6 mice underwent 90 min of ischemia. A group of mice was sacrificed 4 hours after reperfusion for serology, while liver histology was performed on the second group of mice 24 hours after I/R. FK866 did not significantly affect early liver toxicity, as judged by ALT serum levels (Ctrl mice: 32,5 +/- 11 U/ml; solvent-treated, 4 hours after I/R: 1700 +/- 1097 U/ml; FK866-treated, 4 hours after I/R: 1785 +/- 1341 U/ml). The liver histology however, revealed a beneficial effect of FK866 on I/R. Indeed, while large zones of necrosis and mononuclear cell infiltration were observed in the liver lobe that underwent I/R, mice treated with FK866 displayed a marked reduction in both liver necrosis and granuloma formation, suggesting an impaired inflammatory response to I/R in these mice (Figure 1).

## Claims

1. Use of an inhibitor of the formation of NAD for the preparation of a medicament used in the prevention or treatment of ischemia reperfusion injury.
